# EUROPÄISCHE PATENTSCHRIFT

(11) **EP 4 103 150 B1**
(45) Veröffentlichungstag und Bekanntmachung des Hinweises auf die Patenterteilung: **13.09.2023**
(21) Anmeldenummer: 20780989.8
(22) Anmeldetag: 24.09.2020
(51) Int. Cl.: A61K 8/89, A61K 8/894, C08L 83/04

(54) **VERFAHREN ZUR HERSTELLUNG VON HYDROPHILEN ORGANOPOLYSILOXANGELEN**
METHOD FOR PRODUCING HYDROPHILIC ORGANOPOLYSILOXANE GELS
PROCÉDÉ DE PRODUCTION DE GELS D'ORGANOPOLYSILOXANE HYDROPHILES

(43) Veröffentlichungstag der Anmeldung: 21.12.2022
(73) Patentinhaber: Wacker Chemie AG, 81737 München (DE)
(72) Erfinder: SANDMEYER, Frank, 84508 Burgkirchen (DE); BACHMEIER, Bernd-Josef, 84533 Haiming (DE)
(74) Vertreter: Mieskes, Klaus Theoderich
(86) Internationale Anmeldenummer: PCT/EP2020/076786
(87) Internationale Veröffentlichungsnummer: WO 2022/063405

(56) Entgegenhaltungen:
- US-A1- 2016 311 980

## Beschreibung

Die Erfindung betrifft hydrophile Organopolysiloxangele, ein Verfahren zu deren Herstellung sowie deren Verwendung in kosmetischen Zusammensetzungen.

Organopolysiloxangele können durch Vernetzung eines ungesättigten Organopolysiloxans mit einem Si-H-haltigen Organopolysiloxan, im Weiteren auch Si-H-funktioneller Vernetzer genannt, in Gegenwart eines Verdünnungsmittels hergestellt werden.

Vernetzungen sind Verbindungen von Polymerketten in einem dreidimensionalen Netzwerk. Sie können als langkettige Verzweigungen betrachtet werden, die so zahlreich sind, dass ein kontinuierliches unlösliches Netzwerk oder Gel gebildet wird.

Organopolysiloxannetzwerke werden häufig über platinkatalysierte Hydrosilylierungsreaktionen hergestellt. Dabei werden häufig ein Si-H-haltiges Organopolysiloxan und ein Vinyl-funktionelles Organopolysiloxan miteinander zur Reaktion gebracht. Eine wesentliche Voraussetzung für den Aufbau eines 3-dimensionalen Netzwerkes ist dabei, dass mindestens eine der beiden Komponenten, das Si-H-haltige Organopolysiloxan oder das Vinyl-funktionelle Organopolysiloxan, in der mittleren Zusammensetzung mehr als zwei Funktionalitäten pro Molekül aufweist.

Die platinkatalysierte Hydrosilylierungsreaktion bietet bei der Ausbildung von Organopolysiloxannetzwerken den Vorteil, dass keine Nebenprodukte gebildet werden, und dass Verknüpfungsstellen und Netzwerkarchitektur eng definiert sind.

Die wichtigsten Gründe für die Anwendung von Organopolysiloxangelen in kosmetischen Anwendungen sind die dadurch erzielten sensorischen Vorteile, insbesondere die Verbesserung des Hautgefühls von kosmetischen Formulierungen. Darüber hinaus dienen Organopolysiloxangele als Verdickungsmittel in kosmetischen Formulierungen.

US6423322 B1 offenbart Organopolysiloxangele, die durch Hydrosilylierungsreaktion eines speziellen, vinylfunktionellen MQ-Harzes mit einem Si-H-haltigen Organopolysiloxan in Gegenwart eines Verdünnungsmittels und einer kleinen Menge Platin-Hydrosilylierungskatalysator in einem thermischen Prozess, d.h. unter Aufheizen auf eine Temperatur unterhalb des Siedepunktes des verwendeten Verdünnungsmittels leicht hergestellt werden können.

US5654362 lehrt Silicongele für kosmetische Anwendungen, die durch Hydrosilylierungsreaktionen von alpha-omega Dienen mit terminal und oder kettenständig Si-H-funktionellen linearen Polyorganosiloxanen erhalten werden. Die Hydrosilylierung findet unter Verwendung eines geeigneten Katalysators in einem Lösemittel statt, welches selbst ein Polyorganosiloxan sein kann.

US20120202895 A1 lehrt eine pastöse Zubereitung für Kosmetikanwendungen, erhalten durch die Hydrosilylierung von einem oder mehreren Si-H-haltigen Polyorganosiloxanen mit einem oder mehreren aliphatisch ungesättigten Polyorganosiloxanen in einem bei 25°C flüssigen Öl als Lösemittel. In diesem Fall handelt es sich bei den eingesetzten Polyorganosiloxanen ausschließlich um lineare Polyorganosiloxane, nicht um Harze. Mindestens zwei der verwendeten Polyorganosiloxane aus der Gruppe der Si-H-haltigen und ungesättigten linearen Polyorganosiloxane müssen eine Kettenlänge von mehr als 30 Wiederholungseinheiten aufweisen, damit in der Anwendung kein öliges, schmieriges Hautgefühl auftritt.

Diesen Verfahren gemeinsam ist, dass die erhaltenen vernetzten Gelstrukturen ausschließlich hydrophob sind.

US2016311980A1 beschreibt ein Verfahren zur Herstellung von Glycosidresten aufweisenden Organopolysiloxangelen aus einem ungesättigten Polyorganosiloxanharz, einem eine hydrosilylierbare Endgruppe aufweisenden Glycosidrest und einem oder mehreren Si-H-funktionellen Organopolysiloxanen, in einem Verdünnungsmittel in Gegenwart eines Hydrosilylierungskatalysators. Die Si-H-haltigen Vernetzer weisen dabei vorzugsweise niedrige Si-H-Gehalte auf. Sie können hydrophile Substanzen wie Wasser oder Glykole aufnehmen, ohne ihre viskose Gelstruktur zu verlieren. Die Herstellung der erfindungsgemäßen Gele erfolgt vorzugsweise in einem zweistufigen Verfahren, wobei im ersten Schritt die hydrophile Komponente, also das Glykosid und im zweiten Schritt die hydrophobe Komponente, also das Siliconharz hydrosilyliert werden.

US20160317427A1 beschreibt ein Verfahren zur Herstellung von Polyetherresten aufweisenden Organopolysiloxangelen aus einem ungesättigten Polyorganosiloxanharz, polyoxyalkylierten, endständig ungesättigten Alkoholen mit Mischungen Si-H-funktioneller Organopolysiloxane in einem Verdünnungsmittel in Gegenwart eines Hydrosilylierungskatalysators. Die Si-H-haltigen Vernetzer weisen dabei vorzugsweise niedrige Si-H-Gehalte auf. Sie können hydrophile Substanzen wie Wasser oder Glykole aufnehmen ohne ihre viskose Gelstruktur zu verlieren. Die Herstellung der erfindungsgemäßen Gele erfolgt vorzugsweise in einem zweistufigen Verfahren, wobei im ersten Schritt die hydrophile Komponente, also das Polyglykol, und im zweiten Schritt die hydrophobe Komponente, also das Siliconharz, hydrosilyliert werden.

Diesen Verfahren gemeinsam ist, dass die erhaltenen vernetzten Gelstrukturen ausschließlich hydrophil sind.

Will man auf die vorbeschriebene Art und Weise ein hydrophobes Elastomergel oder ein hydrophiles Elastomergel herstellen, so bedingt dies jeweils eine eigene Synthese und das erhaltene Gel hat entweder hydrophile oder hydrophobe Eigenschaften. Auch die Abstufung der Ausprägung der jeweiligen Eigenschaft hydrophil oder hydrophob ist durch die Synthese der jeweiligen Verbindung eindeutig festgelegt.

Aus Gründen der Wirtschaftlichkeit wäre es von Vorteil die Eigenschaften hydrophil oder hydrophob wahlweise durch geeignetes Abmischen entsprechender Komponenten nach Art eines Baukastens einstellen und abstufen zu können. Dabei müssen die übrigen Eigenschaften, die üblicherweise an Elastomergele gestellt werden wie beispielsweise gutes Hautgefühl, Klarheit und Cremigkeit durch eine stabile viskose Gelstruktur sowohl in der hydrophoben als auch der hydrophilen Einstellung der jeweiligen Mischungen erhalten bleiben.

Zudem müssen die Mischungen in einfacher Weise herstellbar sein. Durch Mischen zweier hochviskoser Gele ist diese Anforderung im betrieblichen Alltag nicht erreichbar, denn sie müssen aufgrund ihrer Viskosität entweder manuell vereinigt werden, was weder unter dem Aspekt der Kosten noch unter dem Gesichtspunkt industriell relevanter Produktionsgeschwindigkeiten sinnvoll ist. Man muss sie zueinander pumpen, wobei die Verbindungsleitungen, durch die der Pumpvorgang hindurch stattfindet, anschließend mit erheblichem Aufwand gereinigt werden müssen, denn hochviskose Gele können nicht einfach durch Spülen mit niederviskosen Flüssigkeiten aus Leitungen entfernt werden. Niederviskose Reinigungsflüssigkeiten schaffen sich Kanäle durch hochviskose Gele, durch die sie hindurchfließen ohne das Gel selbst dabei zu entfernen. Mit zunehmend hochviskosen Reinigungsmedien oder durch Abbau der Leitungen und manuelle Reinigung, ist eine Entfernung möglich, was aber ganz offensichtlich wiederum einen erheblichen Aufwand bedeutet und letztendlich gegenüber der Synthese separater Elastomergele einen verschwindenden oder keinen wirtschaftlichen Vorteil mehr bringen würde. Zumal die Reinigungslösungen anschließend zu entsorgen wären. Ein wirtschaftliches Verfahren darf diese Nachteile nicht aufweisen.

US5831080 beschreibt Glycosidreste aufweisende Organosiliziumverbindungen und ein Verfahren zu deren Herstellung.

US20130115184A1 beschreibt neuartige Saccharid Siloxan Copolymere und Verfahren zu deren Herstellung.

Sie werden jeweils als Tenside und Emulgatoren oder zur Verbesserung der Eigenschaften kosmetischer Formulierungen eingesetzt.

US20080199417A1 lehrt Saccharid Siloxane, deren Herstellung und deren Verwendung zur Verbesserung der Eigenschaften kosmetischer Zubereitungen.

Diesen drei Schriften ist gemein, dass sie keine Elastomergele sondern davon verschiedene Siliziumverbindungen sind, und deren Verwendung beispielsweise in Kosmetischen Zusammensetzungen offenbaren, wobei diese Siliziumverbindungen dort bspw. die Funktion als Tenside, Emulgatoren und Schaumstabilisatoren einnehmen.

Es bestand die Aufgabe zur Bereitstellung eines einfachen Baukastensystems, das mit einem minimalen Syntheseaufwand wahlweise die Herstellung hydrophober und hydrophiler Elastomergele für Kosmetikanwendungen gestattet und die oben erwähnten Nachteile nicht aufweist.

Überraschender Weise wurde gefunden, dass die gestellte Aufgabe durch die erfindungsgemäße hydrophile Organopolysiloxangelzubereitung gelöst wird. Sie enthalten
2 bis 80 Gew.-% einer Mischung (X) aus
   - 80 bis 99,9 Gew.-% mindestens eines hydrophoben Organopolysiloxangels hergestellt nach einem in der Beschreibung offenbarten Verfahren,
   - 0,1 bis 20 Gew.-% mindestens einer Verbindung (3) ausgewählt aus der Gruppe enthaltend
      (3a) Glycosidreste aufweisend Organopolysiloxanen der allgemeinen Formel (V),

         RₕR³ᵢSiO_{(4-h-i)/2} (V),
      wobei
      R gleich oder verschieden sein kann und einen einwertigen, gegebenenfalls substituierten Kohlenwasserstoffrest mit 1 bis 18 Kohlenstoffatomen je Rest bedeutet, wobei Heteroatome und -gruppen ausgeschlossen sind, die eine Hydrosilylierungsreaktion durch negative Einwirkung auf den Katalysator behindern würden,
      R³ gleich oder verschieden sein kann und einen Rest der Formel
      Z-(R⁴O)ⱼ-R⁵- (Va) bedeutet, worin
      Z einen Glycosidrest bedeutet, der aus 1 bis 10 Monosaccharideinheiten, vorzugsweise Glucoseeinheiten, aufgebaut ist,
      R⁴ gleich oder verschieden sein kann und einen zweiwertigen Kohlenwasserstoffrest mit 2 bis 4 C-Atomen bedeutet,
      j 0 oder eine ganze Zahl von 1 bis 20 ist und R⁵ gleich oder verschieden sein kann und einen zweiwertigen Kohlenwasserstoffrest mit 2 bis 12 C-Atomen bedeutet,
      h und i jeweils 0, 1, 2 oder 3 bedeuten, mit der Maßgabe, dass h + i ≤ 3 und in mindestens einer Wiederholungseinheit i mindestens den Wert 1 hat;
      (3b) Polyglykolresten aufweisenden Organopolysiloxanen der Formel (VI)

         RₕR⁶ᵢSiO_{(4-h-i)/2} (VI),
      wobei
      R die oben angegebenen Bedeutung hat,
      R⁶ gleich oder verschieden sein kann und einen Rest der Formel

         P-R⁷-R⁸- (VIa)

         bedeutet, worin
         P einen polyoxyalkylierten Rest der Art - (OCₙH₂ₙ)ₘ-OH bedeutet,
         R⁷ eine chemische Bindung oder ein zweiwertiger Kohlenwasserstoffrest mit 1 bis 10 Kohlenstoffatomen, vorzugsweise ein Rest der Formel -CH₂- , -CH(CH₃)- oder -C(CH₃)₂- , bevorzugt ein Rest der Formel -CH₂- , ist und
         n eine ganze Zahl von 1 bis 4, bevorzugt 2 oder 3, ist und
         m eine ganze positive Zahl, vorzugsweise von 1 bis 40, ist,
         R⁸ einen zweiwertigen Kohlenwasserstoffrest mit 2 bis 12 C-Atomen bedeutet, vorzugsweise ein Rest der Formel -CH₂-CH₂-,
         -CH₂-CH(CH₃)- oder -CH₂-C(CH₃)₂-, bevorzugt ein Rest der Formel -CH₂-CH₂- ist,
         h und i jeweils die weiter oben angegebene Bedeutung haben;
      (3c) Cyclodextrinresten aufweisenden Organopolysiloxanen der Formel (VII)

         RₕR⁹ᵢSiO_{(4-h-i)/2} (VII),
      wobei
      R die oben angegebenen Bedeutungen haben kann,
      R⁹ gleich oder verschieden sein kann und einen Rest der Formel

         C-R¹⁰- (VIIa)

         bedeutet, worin
         C einen Cyclodextrinrest der Form 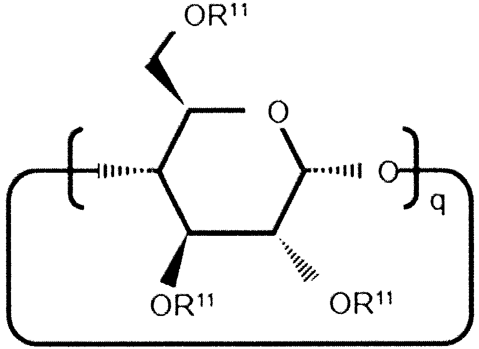
         bedeutet,
         in dem R¹¹ einen Wasserstoffrest oder einen Methylrest bedeutet, und
         q die ganzen Zahlen 6 (alpha Cyclodextrin), 7 (beta Cyclodextrin) oder 8 (gamma Cyclodextrin) bedeutet,
         R¹⁰ einen zweiwertigen gegebenenfalls mit Heteroatomen unterbrochenen Kohlenwasserstoffrest mit 2 bis 18 Kohlenstoffatomen bedeutet,
         h und i jeweils die weiter oben angegebene Bedeutung haben;
         wobei die Summe der Mischung (X) stets 100 Gew.-% ergibt;
und 20 bis 98 Gew.% mindestens eines Verdünnungsmittels (4) zur Viskositätseinstellung, welches verschieden ist von (3a), (3b) und (3c);
wobei die hydrophile Organopolysiloxangelzubereitung eine Steigerung der Wasseraufnahmefähigkeit um mindestens den Faktor 10 aufweist, verglichen mit dem eingesetzten hydrophoben Organopolysiloxangel;
und wobei die Summe aus der Mischung (X) und dem Verdünnungsmittel (4) stets 100 Gew.-% der hydrophilen Organopolysiloxangelzubereitung ergibt.

Dabei sind die Mengen an Verbindung (3), die für die Überführung eines hydrophoben Gels in ein erfindungsgemäßes hydrophiles Gel erforderlich sind überraschend niedrig. Es hat sich gezeigt, dass sich die Zumischung zu großer Mengen an Verbindung (3) schädlich auswirkt, da hierdurch die hochviskose cremige Gelkonsistenz teilweise oder vollständig verloren gehen kann. Details hierzu werden weiter unten in der Detailbeschreibung der Erfindung angegeben und mit Beispielen illustriert.

Der Wechsel der Eigenschaft von hydrophob nach hydrophil wird dabei bestimmt durch die Fähigkeit des erhaltenen Gels Wasser aufzunehmen. Die Fähigkeit zur Wasseraufnahme eines hydrophoben Gels steigt dabei kontinuierlich in dem Maße wie Verbindungen (3) zugesetzt werden. Als signifikant und damit wesentlich für den Eigenschaftswechsel von hydrophob nach hydrophil gilt eine Steigerung der Wasseraufnahmefähigkeit eines hydrophoben Gels um den Faktor 10. Kleinere Wasseraufnahmemengen sind möglich, sind jedoch für die vorliegende Erfindung nicht erfindungsgemäß und sind damit als Gegenstand der Erfindung ausgeschlossen.

Insbesondere ist überraschend, dass die erfindungsgegenständlichen hydrophilen Organopolysiloxangelzubereitungen aus hydrophoben Ausgangsgelen bei gleicher Menge an hydrophilen Gruppen in der Mischung mindestens genauso viel Wasser aufnehmen können wie Elastomergele, die ausschließlich als hydrophile Elastomergele hergestellt wurden, obwohl es sich bei den erfindungsgemäßen hydrophilen Organopolysiloxangelzubereitungen lediglich um physikalische Mischungen eines hydrophoben Elastomergels mit einem hydrophile Gruppen enthaltenden Organopolysiloxan der Verbindung (3) handelt.

### Hydrophobe Organopolysiloxangele (auch als hydrophobe Elastomergele bezeichnet)

Die Mischung (X) enthält 80 bis 99,9 Gew.% hydrophobe Organopolysiloxangele, vorzugsweise 85 bis 99,8 Gew.%, bevorzugt 90 bis 99,7 Gew.%.

Bei den hydrophoben Organopolysiloxangelen handelt es sich um solche, die erhalten werden aus der Umsetzung entweder eines ungesättigten Organopolysiloxanharzes (1a) oder eines ungesättigten Organopolysiloxans (1b) oder eines Diens (1c) mit einem Si-H-haltigen Organopolysiloxan (2a) gegebenenfalls im Gemisch mit einem Si-H-haltigen Organopolysiloxan (2b) in Gegenwart eines die Anlagerung von Si-gebundenem Wasserstoff an aliphatische Mehrfachbindung fördernden Katalysatoren (K) (=Hydrosilylierungskatalysator) in Anwesenheit eines Verdünnungsmittels (4), wobei die Reaktion durch Zusatz einer als Katalysatorgift eingesetzten Stopperverbindung (5) gestoppt wird, die im hydrophoben Organopolysiloxangel verbleibt.

Bei der Auswahl der beiden Si-H-funktionellen Organopolysiloxane sind gegebenenfalls die Erkenntnisse und Rahmenbedingungen gemäß WO2018228657A zu beachten.

Es sind auch solche Gele möglich wie sie beispielsweise in US 6881416B2 beschrieben sind, bei denen im ersten Schritt eine ungesättigte organische Komponente mit nur einer Doppelbindung (1c) hydrosilyliert wird, wobei nur ein Teil der in der Si-H-Gruppen des Si-H-haltigen Organopolysiloxans (2a) gegebenenfalls im Gemisch mit einem Si-H-haltigen Organopolysiloxan (2b) verbraucht werden und die restlichen Si-H-Funktionen für Hydrosilylierungsreaktionen mit einem ungesättigten Organpolysiloxanharz (1a) wie in US6881416B1 oder grundsätzlich auch einem ungesättigten Organopolysiloxan (1b) zur Verfügung stehen.

Es ist hier zu beachten, dass weder die hydrophoben Organopolysiloxangele noch die Verfahren zu deren Herstellung hier erfindungsgegenständlich sind. Sie sind alle dem Durchschnittsfachmann aus dem Stand der Technik bekannt und werden konsequenterweise hier nicht beansprucht. Da sie zur Erläuterung und zum Verständnis der Erfindung aber unverzichtbar sind, werden sie im Folgenden im Detail beschrieben.

Ungesättigte Organopolysiloxanharze (1a) sind aufgebaut aus Einheiten der allgemeinen Formel (I)

RₓR¹_{y}SiO_{(4-x-y)/2} (I)

wobei
R gleich oder verschieden sein kann und einen einwertigen, gegebenenfalls substituierten Kohlenwasserstoffrest mit 1 bis 18 Kohlenstoffatomen je Rest bedeutet, wobei Heteroatome und - gruppen ausgeschlossen sind, die eine Hydrosilylierungsreaktion durch negative Einwirkung auf den Katalysator behindern würden, R¹ einen einwertigen Kohlenwasserstoffrest bedeutet, an den Si-H-Gruppen in einer Hydrosilylierungsreaktion angelagert werden können, vorzugsweise einen einwertigen eine terminale, aliphatische C-C-Mehrfachbindung aufweisenden
Kohlenwasserstoffrest mit 2 bis 18 Kohlenstoffatomen, bevorzugt einen ω-Alkenylrest mit 2 bis 12 C-Atomen, besonders bevorzugt einen Vinylrest bedeutet,
x 0, 1, 2 oder 3 bedeutet,
y 0, 1 oder 2 bedeutet, vorzugsweise 0 oder 1, mit der Maßgabe, dass die Summe x+y kleiner gleich 3 ist, und dass pro Molekül mindestens 2 Reste R', vorzugsweise mindestens 3 Reste R¹, mindestens 20 Mol-% T- und/oder Q-Einheiten (T-Einheiten: Summe x+y=1; Q-Einheiten: Summe x+y=0), vorzugsweise mindestens 20 Mol-% Q-Einheiten, vorhanden sein müssen, und daneben D-Einheiten (Summe x+y=2) vorhanden sein können.

Bevorzugt handelt es sich bei den ungesättigten Organopolysiloxanharzen der Formel (I) um MQ-Harze aus Einheiten der Formeln
SiO₂ (Q-Einheiten) und
R₃SiO_{1/2} und R₂R¹SiO_{1/2} (M-Einheiten),
wobei R und R¹ die oben dafür angegebene Bedeutung haben.
Das molare Verhältnis von M- zu Q-Einheiten liegt dabei vorzugsweise im Bereich von 0,5 bis 4,0, bevorzugt im Bereich von 0,5 bis 2,0, besonders bevorzugt im Bereich von 0,6 bis 1,5. Diese Siliconharze können außerdem bis zu 10 Gew.-% freie Hydroxy- oder Alkoxygruppen enthalten.

Ungesättigte Organopolysiloxane (1b) sind Organopolysiloxane der allgemeinen Formel (II)

R¹_{c}R_{3-c}SiO(R₂SiO)ₐ(R_{2-d}R¹_{d}SiO)_{b}SiR_{3-c}R¹_{c} (II),

wobei
c 0 oder 1, vorzugsweise 1 ist,
d 0 oder 1, vorzugsweise 0 ist,
R und R¹ die oben dafür angegebenen Bedeutungen haben
a und b ganze Zahlen sind,
mit der Maßgabe, dass a + b = 25 - 700, vorzugsweise 30 - 500, insbesondere 40 - 300, vor allem 45 - 160, wobei die Randwerte jeweils mit einbezogen sind, so dass die ungesättigten Organopolysiloxane (1b) vorzugsweise 2 terminal ständige ungesättigte Gruppen enthalten, wobei auch mehr ungesättigte Gruppen enthalten sein können und diese terminal oder kettenständig angeordnet sein können.

Ungesättigte Monoolefine (1c) sind hydrosilylierbare Kohlenwasserstoffe die mindestens 4 C-Atome umfassen, vorzugsweise mindestens 5 C-Atome, besonders bevorzugt mindestens 6 C-Atome, insbesondere mindestens 7 C-Atome. Es ist dabei besonders bevorzugt, dass das Monoolefin (1c) 8 - 24 C-Atome enthält, insbesondere 8 - 20 C-Atome. Die Monoolefine (1c) können linear, verzweigt oder cyclisch sein. Nicht limitierende Beispiele sind 1-Buten, 1-Penten, 2-Buten, 2-Penten, 1-Hexen, 2-Hexen, 3-Hexen, 1-Octen, Isoocten, 1-Decen, 1-Dodecen, Cyclohexen, Methylcyclohexen, Hexylcyclohexen, Norbornen, Camphen, 1-Octadecen, etc.

Si-H-haltige Polyorganosiloxane (2a) sind Si-H funktionelle Organopolysiloxane der allgemeinen Formel (III)

HₑR₃₋ₑSiO(R₂SiO)_{f}(RHSiO)_{g}SiR₃₋ₑHₑ (III),

wobei
e 0 oder 1, vorzugsweise 0 ist,
R die oben dafür angegebene Bedeutung hat,
f und g ganze Zahlen sind,
mit der Maßgabe, dass die Summe f+g 8 bis 1000, vorzugsweise 20 bis 750, ist,
und dass die Organopolysiloxane (2a) Si-gebundenen Wasserstoff in Mengen von 0,010 bis 1,60 Gew.-%, vorzugsweise von 0,010 - 1,30 Gew.-%, bevorzugt von 0,010 - 1,25 Gew.-%, insbesondere von 0,010 - 0,60 Gew.-% enthalten.

Bevorzugte Si-H-haltige Polyorganosiloxane (2a) sind Si-H funktionelle Organopolysiloxane der allgemeinen Formel (III), wobei
e und R die oben angegebene Bedeutung haben
f und g ganze Zahlen sind,
mit der Maßgabe, dass
die Summe f+g 66 bis 248, vorzugsweise 98 bis 248, bevorzugt 118 bis 168 ist,
dass die Organopolysiloxane (2a) Si-gebundenen Wasserstoff in Mengen von 0,011 bis 0,044 Gew.-%, vorzugsweise von 0,019 bis 0,044 Gew.-%, bevorzugt von 0,022 bis 0,032 Gew.-%, enthalten, und dass die Anzahl an Si-H-Gruppen pro Molekül in der durchschnittlichen Zusammensetzung größer 2 und kleiner als 5 ist.

Diese Si-H-haltigen Polyorganosiloxane (2a) sind insbesondere in der Kombination mit ungesättigten Organopolysiloxanharzen (1a) aus Einheiten der allgemeinen Formel (I) bevorzugt, weil aus dem Stand der Technik, z.B. gemäß US2016311980A1 und US20160317427A1 bekannt ist, dass H-Siloxanäquilibrate mit einer eher geringen Zahl an Si-H-Gruppen besonders vorteilhafte Eigenschaften in kosmetischen Anwendungen aufweisen.

Si-H-haltige Organopolysiloxane (2b) sind Si-H funktionelle Organopolysiloxane der allgemeinen Formel (IV)

H_{e'}R_{3-e'}SiO(R₂SiO)_{f'}(RHSiO)_{g'}SiR_{3-e'}H_{e'} (IV),

wobei
e' 0 oder 1, vorzugsweise 0 ist,
R die oben dafür angegebene Bedeutung hat,
f' und g' ganze Zahlen sind,
mit der Maßgabe, dass die Summe f'+ g' 8 bis 248, vorzugsweise 38 bis 248, ist,
und dass die Organopolysiloxane (2b) Si-gebundenen Wasserstoff in Mengen von 0,011 bis 0,35 Gew.-%, vorzugsweise von 0,045 bis 0,156 Gew.-%, enthalten.

Die erfindungsgemäß eingesetzten Si-H-haltigen Organopolysiloxane (2a) haben vorzugsweise eine Viskosität von 3 bis 15000 mm²/s, besonders bevorzugt 20 bis 10000 mm²/s bei 25°C.

Als Katalysator (K) können bei dem erfindungsgemäßen Verfahren die gleichen Katalysatoren eingesetzt werden, die auch bisher zur Förderung der Anlagerung von Si-gebundenem Wasserstoff an aliphatische Mehrfachbindung eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle. Beispiele für solche Katalysatoren sind metallisches und feinverteiltes Platin, das sich auf Trägern wie Siliciumdioxid, Aluminiumoxid oder Aktivkohle befinden kann, Verbindungen oder Komplexe von Platin, wie Platinhalogenide, z. B. PtCl₄, H₂PtCl₆•6H₂O, Na₂PtCl₄•4H₂0, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus H₂PtCl₆•6H₂0 und Cyclohexanon, Platin-Vinyl-siloxankomplexe, wie Platin-1,3-Divinyl-1,1,3,3-tetramethyl-disiloxankomplexe mit oder ohne Gehalt an nachweisbarem anorganisch gebundenen Halogen, Bis-(γ-picolin)-platindichlorid, Trimethylendipyridinplatindichlorid, Dicyclopentadienplatindichlorid, Dimethylsulfoxidethylenplatin-(II)-dichlorid, Cyclooctadien-Platindichlorid, Norbornadien-Platindichlorid, γ-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-0cten gelöstem Platintetrachlorid mit sec.-Butylamin oder Ammonium-Platinkomplexe. Bevorzugte Hydrosilylierungskatalysatoren sind Platinverbindungen, die in einem zur Verwendung in kosmetischen Formulierungen geeigneten Lösungsmittel vorliegen.

Bevorzugt wird der Katalysator (K) in Mengen 1 bis 100 Gew.-ppm (Gewichtsteilen je Million Gewichtsteilen), besonders bevorzugt 2 bis 75 Gew.-ppm, jeweils berechnet als elementares Platin und bezogen auf das Gesamtgewicht der ungesättigten Organopolysiloxanharze (1a), der ungesättigten Organopolysiloxane (1b), gegebenenfalls der ungesättigten organischen Komponenten (1c) der Si-H funktionellen Organopolysiloxane (2a) oder der Mischung der Si-H funktionellen Organopolysiloxane (2a) mit (2b) und dem Verdünnungsmittel (4).

Vorzugsweise haben die ungesättigten Organopolysiloxanharze (1a) eine Viskosität größer als 0,7 mm²/s bei 25°C. Bevorzugt sind solche Organopolysiloxanharze, die eine Viskosität von größer als 1000 mm²/s bei 25°C haben, oder die Feststoffe sind. Das mit Gelpermeationschromatografie bestimmte gewichtsmittlere Molekulargewicht M_{w} (bezogen auf einen Polystyrolstandard) dieser Organopolysiloxanharze beträgt vorzugsweise 334 bis 200 000 g/mol, bevorzugt 1 000 bis 20 000 g/mol.

Die ungesättigten Organopolysiloxanharze (1a) haben vorzugsweise eine Jodzahl kleiner 254, bevorzugt eine Jodzahl kleiner 76.

Beispiele für Reste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für substituierte Reste R sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorphenylrest.

Bevorzugt handelt es sich bei dem Rest R um einen einwertigen Kohlenwasserstoffrest mit 1 bis 6 Kohlenstoffatomen, wobei der Methylrest besonders bevorzugt ist.

Der ungesättigte Kohlenwasserstoffrest R¹ ist vorzugsweise gebunden an eine M-Einheit (=Mvi) oder D-Einheit (=D_{Vi}), bevorzugt an eine M-Einheit, wobei das molare Verhältnis M: (M_{Vi}+D_{Vi}), vorzugsweise M:Mvi, vorzugsweise im Bereich 0 bis 50, bevorzugt im Bereich 0 bis 20, besonders bevorzugt im Bereich 2,5 bis 13 liegt.

Beispiele für Reste R¹ sind Alkenylreste, wie der Vinyl-, 5-Hexenyl-, Cyclohexenyl-, 1-Propenyl-, Allyl-, 3-Butenyl- und 4-Pentenylrest, und Alkinylreste, wie der Ethinyl-, Propargyl-und 1-Propinylrest. Bevorzugt handelt es sich bei dem Rest R¹ um Alkenylreste, besonders bevorzugt ω-Alkenylreste, insbesondere um den Vinylrest.

Die Herstellung der hydrophoben Gele ist leicht durchführbar. Im Allgemeinen gibt man alle Bestandteile außer dem Katalysator zusammen, rührt langsam, bis sich das gegebenenfalls verwendete feste ungesättigte Organopolysiloxanharz gelöst hat, bzw. bis sich alle Komponenten homogen vermischt haben und setzt dann den Katalysator zu. Die Zusammensetzung kann bei Raumtemperatur belassen werden, bis sich ein Gel gebildet hat, oder falls erforderlich erhitzt werden. Zusammensetzungen, die ungesättigte Organopolysiloxanharze enthalten, werden vorzugsweise auf eine Temperatur zwischen 50°C und 130°C, bevorzugt zwischen 70°C und 120°C erhitzt, bis die Mischung geliert oder fest wird. Ggf. gilt das Gleiche für Zusammensetzungen, die ungesättigte Organopolysiloxane enthalten, deren Reaktivität der von ungesättigten Organopolysiloxanharze vergleichbar ist. Die Gelierung erfolgt vorzugsweise innerhalb von zehn Stunden, bevorzugt innerhalb von drei Stunden. Es werden Organopolysiloxangele erhalten, welche für die Anwendung in kosmetischen Formulierungen geeignet sind.

Zur Herstellung der hydrophoben Organopolysiloxangele werden ungesättigte Organopolysiloxanharze (1a) oder ungesättigte Organopolysiloxane (1b) in Mengen von vorzugsweise 4,5 bis 0,1 Mol, bevorzugt 2 bis 0,3 Mol, besonders bevorzugt 1,8 bis 0,5 Mol, Kohlenwasserstoffrest mit aliphatischer C-C-Mehrfachbindung je Mol Si-gebundenem Wasserstoff in den Si-H-haltigen Organopolysiloxanen (2a) und ggf. (2b) eingesetzt.

Die Reaktion wird am Ende der Reaktionszeit durch Zugabe vorzugsweise eines Hydrosilylierungskatalysatorgiftes als Stopperverbindung (5) abgebrochen, wodurch die Nachhärtung, die durch verbleibende vernetzende Hydrosilylierungsreaktionen, die in den Siliconelastomeren auftreten, bewirkt wird, beendet wird. Als Stopper werden dabei Katalysatorgifte eingesetzt, die den Hydrosilylierungskatalysator irreversibel verändern und dadurch deaktivieren.

Die Stopperverbindungen (5) werden vorzugsweise in Mengen von mindestens 1,1 Mol die Stopperung bewirkende funktionelle Gruppe, vorzugsweise Mercaptogruppe, je Mol elementares Platin, im Katalysator (K) eingesetzt.

Es hat sich dabei als besonders vorteilhaft erwiesen, eine deutlich überstöchiometrische Menge des Stoppers einzusetzen, da dies eine positive Wirkung auf den Langzeiterhalt der Cremigkeit des fertigen Gels hat.

Beispielhaft für Stopperverbindungen (5), welche geeignet sind, um die Nachhärtung zu beenden, sind schwefelorganische Verbindungen, wie Mercaptogruppen aufweisende organische Verbindungen. Weitere geeignete Verbindungen sind in

US 6,200,581 genannt. Bevorzugte Hydrosilylierungskatalysatorgifte als Stopperverbindungen (5) sind Mercaptoalkylgruppen aufweisende Organopolysiloxane, besonders bevorzugt sind 3-Mercaptopropylgruppen aufweisende Organopolysiloxane, wie mercaptopropylfunktionelle Silsesquisiloxane oder mercaptopropylfunktionelle Polyorganosiloxane.

Als Stopperverbindungen (5) eingesetzte Mercaptoalkylgruppen aufweisende Organopolysiloxane werden vorzugsweise in Mengen von 200 bis 1,1 Mol, bevorzugt 50 bis 1,5 Mol, besonders bevorzugt 20 bis 2,0 Mol, Mercaptogruppen je Mol elementares Platin im Katalysator (K) eingesetzt.

Optional kann je nach gewähltem Verfahren in einem zweiten oder dritten optionalen Verfahrensschritt eine Verdünnung des zunächst erhaltenen hydrophoben Organopolysiloxanbasisgels erfolgen.

In dem optionalen Verdünnungsschritt des hydrophoben Organopolysiloxanbasisgels ist es möglich, eine Vielzahl unterschiedlicher hydrophober Gele herzustellen, die in ihrer Konsistenz und ihrem Eigenschaftsprofil in einem breiten Bereich variieren. Man kann dabei das gleiche Verdünnungsmittel (4) verwenden, welches im ersten bzw. den ersten beiden Verfahrensschritten verwendet worden ist, oder ein zweites Verdünnungsmittel (4), das hiervon verschieden ist.

### Verbindung (3)

Bei den Glycosidreste enthaltenden Organopolysiloxanen (3a) handelt es sich um solche der Formel (V)

RₕR³ᵢSiO_{(4-h-i)/2} (V),

wobei
R die oben angegebenen Bedeutungen haben kann,
R³ gleich oder verschieden sein kann und einen Rest der Formel
Z-(R⁴O)ⱼ-R⁵- (Va) bedeutet, worin
Z einen Glycosidrest bedeutet, der aus 1 bis 10 Monosaccharideinheiten, vorzugsweise Glucoseeinheiten, aufgebaut ist,
R⁴ gleich oder verschieden sein kann und einen zweiwertigen Kohlenwasserstoffrest mit 2 bis 4 C-Atomen bedeutet,
j 0 oder eine ganze Zahl von 1 bis 20 ist und
R⁵ gleich oder verschieden sein kann und einen zweiwertigen Kohlenwasserstoffrest mit 2 bis 12 C-Atomen bedeutet,
h und i jeweils 0, 1, 2 oder 3 bedeuten, mit der Maßgabe, dass h + i ≤ 3 und in mindestens einer Wiederholungseinheit i mindestens den Wert 1 hat.

Beispiele für Reste der Formel (Va) sind

G- (CH₂CH₂O)-CH₂CH₂-,

G- (CH₂CH₂O)-CH₂CH₂CH₂-,

G₂-(CH₂CH₂O)-CH₂CH₂-,

G₂-(CH₂CH₂O)-CH₂CH₂CH₂-,

wobei G einen Glycosidrest der Formel (C₆H₁₁O₆)- und
G₂ einen aus zwei Glucoseeinheiten aufgebauten Glycosidrest bedeutet.

Bevorzugte Beispiele für Reste der Formel (Va) sind G-(CH₂CH₂O)-CH₂CH₂CH₂- und G₂-(CH₂CH₂O)-CH₂CH₂CH₂-, wobei G und G₂ die oben dafür angegebene Bedeutung haben.

Bevorzugte Glucosidreste enthaltende Polyorganosiloxane der Formel (V) sind solche, die einen HLB-Wert aufweisen von 3 bis 18, besonders von 4 bis 16, insbesondere von 4 bis 12. ganz besonders vorteilhaft haben sich HLB Werte von 5 bis 10 erwiesen.

Bei den Polyglycolreste enthaltenden Organopolysiloxanen (3b) handelt es sich um solche der Formel (VI)

RₕR⁶ᵢSiO_{(4-h-i)/2} (VI),

wobei
R die oben angegebenen Bedeutungen haben kann,
R⁶ gleich oder verschieden sein kann und einen Rest der Formel

   P-R⁷-R⁸- (VIa)

   bedeutet, worin
   P einen polyoxyalkylierten Rest der Art -(OCₙH₂ₙ)ₘ-OH bedeutet,
   R⁷ eine chemische Bindung oder ein zweiwertiger Kohlenwasserstoffrest mit 1 bis 10 Kohlenstoffatomen, vorzugsweise ein Rest der Formel -CH₂- , -CH(CH₃)- oder -C(CH₃)₂- , bevorzugt ein Rest der Formel - CH₂- , ist und
   n eine ganze Zahl von 1 bis 4, bevorzugt 2 oder 3, ist und m eine ganze positive Zahl, vorzugsweise von 1 bis 40, ist, R⁸ einen zweiwertigen Kohlenwasserstoffrest mit 2 bis 12 C-Atomen bedeutet, vorzugsweise ein Rest der Formel -CH₂-CH₂-, -CH₂-CH(CH₃)- oder -CH₂-C(CH₃)₂-, bevorzugt ein Rest der Formel -CH₂-CH₂- ist,
h und i jeweils die weiter oben angegebene Bedeutung haben.

Bevorzugte Polyglycolreste enthaltende Polyorganosiloxane der Formel (VI) sind solche, die einen HLB-Wert aufweisen von 3 bis 18, besonders von 4 bis 16, insbesondere von 4 bis 12. ganz besonders vorteilhaft haben sich HLB Werte von 5 bis 10 erwiesen.

Bevorzugte Beispiele für polyoxyalkylierte Reste P sind solche der allgemeinen Formel

- (OCH₂CH₂)ₒ[OCH₂CH(CH₃)]ₚ-OH (VIb),

wobei
o 0 oder eine ganze Zahl von 1 bis 30, vorzugsweise 2 bis 20, bevorzugt 6 bis 14 ist und
p 0 oder eine ganze Zahl von 1 bis 30, vorzugsweise 0 bis 10, bevorzugt 0 ist,
wobei die Summe von o+p 1 bis 40, vorzugsweise 2 bis 20, bevorzugt 6-14 ist.

Ein besonders bevorzugtes Beispiel für einen polyoxyalkylierten, Rest P ist der Polyethylenglykolrest mit etwa 10 Oxyethylen-Einheiten.

Die Formel (VIb) soll dabei so verstanden werden, dass o Gruppen -(OCH₂CH₂)- und p Gruppen -[OCH₂CH(CH₃)] - in beliebiger Weise im Rest P verteilt sein können.

Bei den Cyclodextrinreste enthaltenden Organopolysiloxanen (3c) handelt es sich um solche der Formel (VII)

RₕR⁹ᵢSiO_{(4-h-i)/2} (VII),

wobei
R die oben angegebenen Bedeutungen haben kann,
R⁹ gleich oder verschieden sein kann und einen Rest der Formel

   C-R¹⁰- (VIIa)

   bedeutet, worin
   C einen Cyclodextrinrest der Form 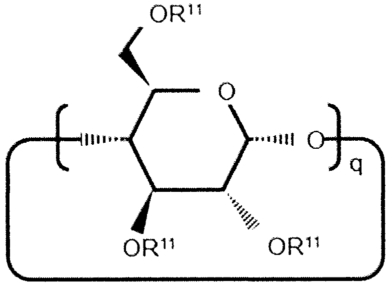 bedeutet,
   in dem R¹¹ einen Wasserstoffrest oder einen Methylrest bedeutet, und
   q die ganzen Zahlen 6 (alpha Cyclodextrin), 7 (beta Cyclodextrin) oder 8 (gamma Cyclodextrin) bedeutet,
   R¹⁰ einen zweiwertigen gegebenenfalls mit Heteroatomen unterbrochenen Kohlenwasserstoffrest mit 2 bis 18 Kohlenstoffatomen bedeutet,
h und i jeweils die weiter oben angegebene Bedeutung haben.

Nicht einschränkende Beispiele für Reste R¹⁰ sind Reste der Form -CH₂-CH₂-, -CH₂-CH(CH₃)- oder -CH₂-C(CH₃)₂-, -CH₂CH₂CH₂-, - CH₂(CH₂)₂CH₂-, -CH₂(CH₂)₃CH₂-, -CH₂(CH₂)₄CH₂-, -CH₂(CH₂)₆CH₂-, - CH₂(CH₂)₁₀CH₂-, -CH₂-CH(CH₃)₂CH₂-, wobei die Reste -CH₂(CH₂)₆CH₂-und -CH₂(CH₂)₁₀CH₂- besonders bevorzugt sind.

Bevorzugte Cyclodextrinreste enthaltende Polyorganosiloxane der Formel (VII) sind solche, die einen HLB-Wert aufweisen von 3 bis 18, besonders von 4 bis 16, insbesondere von 4 bis 12. ganz besonders vorteilhaft haben sich HLB Werte von 5 bis 10 erwiesen.

Hergestellt werden die Organolpolysiloxane (V), die einen Glycosidrest aufweisen, die Organopolysiloxane (VI), die einen Polyoxyalkylenrest aufweisen und die Organopolysiloxane (VII), die einen Cyclodextrinrest aufweisen durch Reaktion einer Si-H-Funktionen enthaltenden Organosiliziumverbindung der Formel

RₕHᵢSiO_{(4-h-i)/2} (IX),

in welcher R, h und i die oben genannten Bedeutungen haben und in der mindestens in einer Wiederholungseinheit i mindestens den Wert 1 bedeutet,
mit ungesättigten Verbindungen der Formel

   Z-(R⁴O)ⱼ-R¹² (Vb),
worin Z, R⁴ und j die oben genannten Bedeutungen haben und
R¹² einen einwertigen Kohlenwasserstoffrest mit 2 bis 12 C-Atomen, an den Si-H-Gruppen in einer Hydrosilylierungsreaktion angelagert werden können, vorzugsweise einen einwertigen eine terminale aliphatische C-C-Mehrfachbindung aufweisenden Kohlenwasserstoffrest mit 3 bis 17 C-Atomen, bevorzugt 4 bis 16 C-Atomen, besonders bevorzugt einen Allylrest bedeutet,
bzw. mit ungesättigten Verbindungen der Formel

   P-R⁷-R¹³ (VIb)
wobei P und R⁷ die oben angegebenen Bedeutungen haben und
R¹³ einen einwertigen Kohlenwasserstoffrest mit 2 bis 12 C-Atomen, an den Si-H-Gruppen in einer Hydrosilylierungsreaktion angelagert werden können, vorzugsweise einen einwertigen eine terminale aliphatische C-C-Mehrfachbindung aufweisenden Kohlenwasserstoffrest mit 2 bis 12 C-Atomen, bevorzugt 3 bis 12 C-Atomen, besonders bevorzugt einen Vinylrest oder einen Allylrest bedeutet,
bzw. mit einer ungesättigten Verbindung der Formel

   C-R¹⁴ (VIIb)
in der C die oben angegebenen Bedeutungen hat und
R¹⁴ einen einwertigen Kohlenwasserstoffrest mit 2 bis 18 C-Atomen, an den Si-H-Gruppen in einer Hydrosilylierungsreaktion angelagert werden können, vorzugsweise einen einwertigen eine terminale aliphatische C-C-Mehrfachbindung aufweisenden Kohlenwasserstoffrest mit 3 bis 17 C-Atomen, bevorzugt 4 bis 16 C-Atomen, besonders bevorzugt einen Octenylrest oder einen Dodecenylrest bedeutet.

In einer bevorzugten Ausführungsform der Erfindung sind die Organosiliziumverbindungen der Formel (IX) Si-H-haltige Polyorganosiloxane der allgemeinen Formel

HₑR₃₋ₑSiO(R₂SiO)_{f}(RHSiO)_{g}SiR₃₋ₑHₑ (III),

die weiter oben bereits beschrieben ist, wobei die Zusammensetzung der Organopolysiloxane der Formel (III) zur Herstellung der Organopolysiloxane der Formeln (V), (VI) und (VII) unabhängig ist von der Zusammensetzung der Organopolysiloxane der Formel (III) zur Herstellung der hydrophoben Organolysiloxangele. Insbesondere können die beiden Organopolysiloxane der Formel (III) für die verschiedenen Einsatzzwecke voneinander verschieden sein und sind dies in der Regel auch.

Die sich daraus ergebenden Strukturen der Organopolysiloxane der Formeln (V), (VI) und (VII) sind dem Fachmann aus den hier gemachten Angaben offensichtlich.

Eine besonders bevorzugte ungesättigte Verbindungen (Vb) sind dabei Alkenylglucoside (Vc), besonders bevorzugt das (2-Allyloxy-ethoxy)-glucosid der Formel

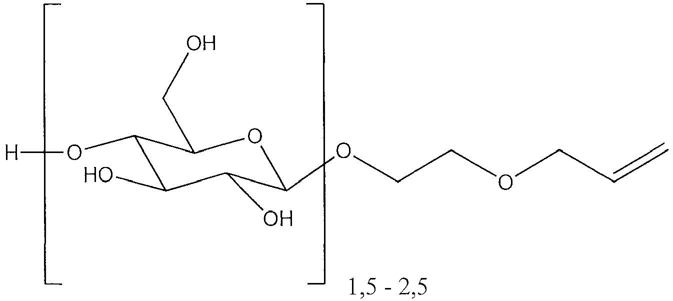

Dieses Alkenylglucosid wird nach dem in US 5,831,080 in Beispiel 1 (A) (Spalte 8, Zeilen 23 - 43) beschriebenem Verfahren hergestellt, mit dem Unterschied, dass das Lösemittel im letzten Schritt nicht vollständig abdestilliert wird, da dies zu einem bei Raumtemperatur glasartigen Material führen würde, sondern dass ein Lösemitteltausch auf 1,2-Propandiol durchgeführt wird. Das Produkt ist eine ca. 50%-ige Lösung des Alkenylglucosids in 1,2-Propandiol. Das Alkenylglucosid liegt teilweise aufkondensiert als Oligoglucosid vor. Die Verbindungen (Vc) werden daher vorzugsweise gelöst in einem organischen Lösungsmittel eingesetzt. Beispiele für organische Lösungsmittel sind Alkohole, wie Glycerin, 1,2-Propandiol, Methanol, Ethanol, n-Propanol und iso-Propanol; gesättigte Kohlenwasserstoffe, wie n-Pentan, n-Hexan, n-Heptan und n-Octan, und deren verzweigte Isomeren, wie Isododecan; Benzine, z. B. Alkangemische mit einem Siedebereich von 80°C bis 140°C bei 1020 hPa; ungesättigte Kohlenwasserstoffe wie 1-Hexen, 1-Hepten, 1-Octen und 1-Decen; aromatische Kohlenwasserstoffe wie Benzol, Toluol und Xylole; halogenierte Alkane mit 1 bis 6 Kohlenstoffatom(en), wie Methylenchlorid, Trichlorethylen und Perchlorethylen; Ether, wie Di-n-butyl-ether; Ester, wie Ethylacetat, Isopropylpalmitat und Isopropylmyristat (=Myristinsäure-isopropylester); Ketone, wie Methylethylketon und Cyclohexanon.

Die Mischung (X) enthält 0,1 bis 20 Gew.-% der Verbindung (3) vorzugsweise 0,2 bis 15 Gew.-%, besonders bevorzugt 0,3 bis 10 Gew.-%.

### Verdünnungsmittel (4)

Verdünnungsmittel (4) sind von (3a), (3b) und (3c) verschieden. Sie sind vorzugsweise Organopolysiloxane mit 2 bis 200 Si-Atomen, bevorzugt Organopolysiloxane mit 2 bis 50 Si-Atomen, besonders bevorzugt lineare Organopolysiloxane mit einer Viskosität von 1,5 bis 50 mm2/s bei 25°C, oder organische Verdünnungsmittel oder Mischungen von Organopolysiloxanen mit 2 bis 200 Si-Atomen mit organischen Verdünnungsmitteln.

100 Gew.-% der erfindungsgemäßen hydrophilen Organopolysiloxangelzubereitung enthält 20 bis 98 Gew.%, Verdünnungsmittel (4), vorzugsweise 30 bis 98 Gew.-%, bevorzugt 50 bis 90 Gew.-%, besonders bevorzugt 60 bis 85 Gew.-%, und 2 bis 80 Gew.-%, vorzugsweise 2 bis 70 Gew.-%, bevorzugt 10 bis 50 Gew.-%, besonders bevorzugt 15 bis 40 Gew.-% der Mischung (X).

Nicht-reaktive oder relativ nicht-reaktive Verdünnungsmittel (4) sind bevorzugt. Im Rahmen der vorliegenden Erfindung wird der Begriff "nicht-reaktiv" in Bezug auf die in Frage stehende Vernetzungsreaktion und die hierin eingesetzten Reaktanden verwendet. Ein relativ nichtreaktives Verdünnungsmittel (4) ist weniger als ein Zehntel so reaktiv mit den Reaktanden der Vernetzungsreaktion im Vergleich zu den Reaktanden untereinander in der Vernetzungsreaktion.

Geeignete Beispiele von Verdünnungsmittel (4) beinhalten cyclische und lineare Organopolysiloxane, organische Verdünnungsmittel oder Mischungen von Organopolysiloxanen und organischen Verdünnungsmitteln.

Das Organopolysiloxan kann ein einzelnes Organopolysiloxan oder eine Mischung von Organopolysiloxanen sein. Das Organopolysiloxan kann Alkyl-, Aryl-, Alkaryl- und Aralkylgruppen tragen. Solche Organopolysiloxane können beispielhaft durch Polydimethylsiloxan, Polydiethylsiloxan, Polymethylethylsiloxan, Polymethylphenylsiloxan und Polydiphenylsiloxan angegeben werden, sind aber nicht darauf beschränkt.

Cyclische Polydimethylsiloxane können beispielhaft durch Hexamethylcyclotrisiloxan, Octamethylcyclotetrasiloxan, Decamethylcyclopentasiloxan und Dodecamethylcyclohexasiloxan angegeben werden, sind aber nicht darauf beschränkt.

Möglich ist auch die Verwendung von funktionellen Organopolysiloxanen als Verdünnungsmittel (4), beispielsweise acrylamidfunktionelle Siloxanfluide, acrylfunktionelle Siloxanfluide, amidfunktionelle Siloxanfluide, aminofunktionelle Siloxanfluide, carbinolfunktionelle Siloxanfluide, carboxyfunktionelle Siloxanfluide, chloralkylfunktionelle Siloxanfluide, epoxfunktionelle Siloxanfluide, glykolfunktionelle Siloxanfluide, ketalfunktionelle Siloxanfluide, mercaptofunktionelle Siloxanfluide, methylesterfunktionelle Siloxanfluide, perfluorofunktionelle Siloxanfluide und silanolfunktionelle Siloxane.

Vorzugsweise ist das Organopolysiloxan ein Polydimethylsiloxan mit 2 bis 200 Si-Atomen, bevorzugt 2 bis 50 Si-Atomen, besonders bevorzugt sind lineare Polydimethylsiloxane mit einer Viskosität von 1,5 bis 50 mm²/s bei 25°C.

Als organische Verdünnungsmittel (4) können aliphatische Kohlenwasserstoffe verwendet werden, wie beispielsweise wie Pentan, Cyclohexan, Heptan, Lackbenzine, Fettöle einschließlich mehrfach ungesättigter ω-3- und ω-6-Fettsäuren, und deren Ester; pflanzliche Öle, wie Erdnuss-, Oliven-, Palm-, Canola-, Maiskeim-, Soja-, Sonnenblumenöl und dergleichen; und natürliche und synthetische Öle oder öllösliche Feststoffe, wie verschiedene Mono-, Di- und Triglyceride, polyoxyalkylierte pflanzliche Öle, Lanolin, Lecithin und dergleichen; und Erdölkohlenwasserstoffe, wie Petrolatum, Mineralöl, Benzin, Petrolether. Diese Beispiele dienen der Erläuterung und sind nicht als Einschränkung zu verstehen.

Das organische Verdünnungsmittel (4) kann auch aliphatische oder alicyclische Kohlenwasserstoffe mit 4 bis 30 C-Atomen, vorzugsweise gesättigte Kohlenwasserstoffe, umfassen. Die aliphatischen Kohlenwasserstoffe können geradkettig oder verzweigt sein, und die alicyclischen Kohlenwasserstoffe können unsubstituierte cyclische Kohlenwasserstoffe oder aliphatische kohlenwasserstoffsubstituierte Kohlenwasserstoffe darstellen. Beispiele für geeignete Kohlenwasserstoffe sind n-Heptan, n-Octan, Isooctan, n-Decan, Isodecan, n-Dodecan, Isododecan, Cyclohexan, Cycloheptan, Cyclooctan, Methylcyclohexan, Dimethylcyclohexan, Ethylcyclohexan, Nonylcyclohexan und dergleichen. Auch diese Aufzählung dient der Erläuterung und ist nicht als Einschränkung zu verstehen.

Geeignete organische Verdünnungsmittel (4) sind auch flüchtige Aromastoffe, wie Pfefferminzöl, Öl von grüner Minze, Menthol, Vanille, Zimtöl, Nelkenöl, Lorbeeröl, Anisöl, Eukalyptusöl, Thymianöl, Zedernöl, Öl von der Muskatnuss, Öl von Salbei, Kassiaöl, Kakao, Süßholzsaft, Stärkezuckersirup aus Mais mit hohem Fructosegehalt, Citrusöle, wie Zitrone, Orange, Limone und Grapefruit, Fruchtessenzen, wie Apfel, Birne, Pfirsich, Traube, Erdbeere, Himbeere, Kirsche, Pflaume, Ananas und Aprikose; und andere nützliche Aromastoffe einschließlich Aldehyde und Ester, wie Zimtsäureessigester, Zimtaldehyd, Eugenylformat, p-Methylanisol, Acetaldehyd, Benzaldehyd, Anisaldehyd, Citral, Neral, Decanal, Vanillin, Tolylaldehyd, 2,6-Dimethyloctanal und 2-Ethylbutyraldehyd.

Ein Teil oder das gesamte organische Verdünnungsmittel (4) kann ein oder mehrere flüchtige Duftstoffe umfassen, wie natürliche Produkte und Parfumöle. Einige stellvertretende natürliche Produkte und Parfumöle sind Amber, Benzoin, Zibet, Nelke, Zedernöl, Jasmin, Mate, Mimose, Moschus, Myrrhe, Iris, Sandelholzöl und Vetiveröl; Aromachemikalien, wie Amylsalicylat, Amylzimtaldehyd, Benzylacetat, Citronellol, Cumarin, Geraniol, Isobornylacetat, Ambrette und Terpinylacetat, und verschiedene klassische Parfümölfamilien, wie die Blumenbouquetfamilie, die orientalische Familie, die Chyprefamilie, die Holzfamilie, die Citrusfamilie, die Canoefamilie, die Lederfamilie, die Gewürzfamilie und die Kräuterfamilie.

Bevorzugte organische Verdünnungsmittel (4) weisen eine Viskosität im Bereich von 0,5 bis 200 mm²/s (25°C) auf, wobei solche Verdünnungsmittel mit einen Siedepunkt im Bereich von 50°C bis 300°C besonders bevorzugt sind.

Es können zahlreiche Mischungen von Verdünnungsmittel (4) verwenden werden, die lediglich auf diejenigen Zusammensetzungen beschränkt sind, bei denen nach der Herstellung der erfindungsgemäßen hydrophilen Organopolysiloxangelzubereitung keine Phasentrennung auftritt.

Die erfindungsgemäßen hydrophilen Organopolysiloxangelzubereitungen weisen ein exzellentes Hautgefühl auf.

### Aktiver Wirkstoff für die Körper- oder Gesundheitspflege

Daher sind ein weiterer Gengenstand der vorliegenden Erfindungen kosmetische Zubereitungen oder Zubereitungen für die Körper- und Gesundheitspflege, Wasch- und Reinigungszubereitungen, Zubereitungen zur langanhaltenden Beduftung oder Insektenabwehr. Solche Zubereitungen enthaltend die erfindungsgemäßen hydrophilen Organopolysiloxangelzubereitungen. Sie enthalten weiterhin in diesem Gebiet allgemein bekannte Zuschlagstoffe sowie beispielsweise "aktive Wirkstoffe für die Körperpflege oder Gesundheitspflege". Dies bedeutet im vorliegenden Zusammenhang eine beliebige Verbindung oder Mischung von Verbindungen, die dem Fachmann im Fachgebiet als Zusatzstoffe in Körperpflegeformulierungen bekannt sind und die typischerweise zugesetzt werden, um das Haar oder die Haut zu behandeln, um einen kosmetischen und/oder ästhetischen Nutzen zu erzielen; eine beliebige Verbindung oder Mischung von Verbindungen, die im Fachgebiet bekannt sind, um einen pharmazeutischen oder medizinischen Nutzen zu erzielen; eine beliebige Verbindung, mit der eine pharmakologische Wirksamkeit oder ein sonstiger Effekt bei der Diagnose, Heilung, Linderung, Behandlung oder Vorbeugung von Krankheiten erzielt werden soll, oder um die Struktur oder eine beliebige Funktion des Körpers eines Menschen oder eines Tiers zu beeinflussen; und jede beliebige Verbindung, die bei der Herstellung von Arzneimittelprodukten eine chemische Veränderung erfahren kann und die in Arzneimitteln in modifizierter Form vorliegen kann, um die angegebene Wirksamkeit oder den angegebenen Effekt hervorzurufen.

Die aktiven Wirkstoffe für die Körperpflege oder Gesundheitspflege sind vorzugsweise ausgewählt aus der Gruppe der fett-oder öllöslichen Vitamine, öllöslichen Arzneimittel, wobei Antiaknemittel, antibakterielle Mittel, fungizide Mittel, entzündungshemmende Mittel, schuppenflechtebekämpfende Mittel, Betäubungsmittel, juckreizlindernde Mittel, hautentzündungshemmende Mittel und Mittel, die im Allgemeinen als Sperrfilme betrachtet werden, und öllöslichen UV-Absorber.

Beispiele für nützliche aktive Bestandteile zur Verwendung im ggf. dritten Verfahrensschritt gemäß der Erfindung sind folgende:
Nichtbeschränkende Beispiele für öllösliche Vitamine sind, Vitamin A₁, RETINOL, C₂- bis C₁₈-Ester von RETINOL, Vitamin E, TOCOPHEROL, Ester von Vitamin E und Mischungen derselben. RETINOL umfasst trans-RETINOL, 13-cis-RETINOL, 11-cis-RETINOL, 9-cis-RETINOL und 3,4-Didehydro-RETINOl.

Es sollte bemerkt werden, dass RETINOL ein International Nomenclature Cosmetic Ingredient Name (INCI), vergeben von The Cosmetic, Toiletry and Fragrance Association (CTFA), Washington DC, für Vitamin A ist. Andere geeignete Vitamine und die INCI-Namen für die in Betracht stehenden Vitamine, die hierin umfasst sind, sind RETINYL ACETAT, RETINYL PALMITATE, RETINYL PROPIONATE, a- TOCOPHEROL,TOCOPHERSOLAN,TOCOPHERYL ACETATE, TOCOPHERYLLINOLEATE, TOCOPHERYLNICOTINATE und TOCOPHERYL SUCCINATE.

Einige Beispiele für kommerziell erhältliche Produkte, die zur Verwendung hierin geeignet sind, sind Vitamin-A-Acetat von Fluka Chemie AG, Buchs, Schweiz; CIOVI-OX T-50, ein Vitamin E-Produkt von Henkel Corporation, La Grange, Illinois; COVI-OX T-70, ein anderes Vitamin-E-Produkt von Henkel Corporation, La Grange, Illinois, und Vitamin-E-Acetat, ein Produkt von Roche Vitamins & Fine Chemicals, Nutley, New Jersey.

Stellvertretende Beispiele für einige geeignete öllösliche Arzneimittel, welche als aktive Bestandteile zugefügt werden können, sind Clonidin, Scopolamin, Propranolol, Estradiol, Phenylpropanolaminhydrochlorid, Ouabain, Atropin, Haloperidol, Isosorbid, Nitroglycerin, Ibuprofen, Ubichinone, Indomethacin, Prostaglandine, Naproxen, Salbutamol, Guanabenz, Labetalol, Pheniramin, Metrifonat und Steroide.

Ebenso hierin umfasst als ein Arzneimittel für die Zwecke der vorliegenden Erfindung sind Antiaknemittel, wie Benzoylperoxid, Triclosan und Tretinoin; antibakterielle Mittel wie Chlorhexidingluconat; fungizide Mittel, wie Miconazolnitrat; entzündungshemmende Mittel, wie Salicylsäure; corticosteroide Arzneimittel; nichtsteroide entzündungshemmende Mittel, wie Diclofenac; schuppenflechtebekämpfende Mittel, wie Clobetasolpropionat und Retinoide, Betäubungsmittel, wie Lidocain; juckreizlindernde Mittel, wie Polidocanol; hautentzündungshemmende Mittel, wie Prednisolon und Mittel, die im Allgemeinen als Sperrfilme betrachtet werden.

Stellvertretende Beispiele für öllösliche UV-Absorber, welche als aktive Bestandteile zugefügt werden können, sind 1-(4-Methoxyphenyl)-3-(4-tert-butylphenyl)propan-1,3-dione (INCI: Butyl Methoxydibenzoylmethan), 2-Ethylhexyl-(2E)-3-(4-methoxyphenyl)prop-2-enoat (INCI: Octyl Methoxycinnamat), 4-Hydroxy-2-methoxy-5-(oxo-phenylmethyl)benzenesulfonic acid (INCI: Benzophenon-4), 2-Hydroxy-4-methoxybenzophenon-5-sulfonsäure Natriumsalz (INCI: Benzophenon-5) und 2-Ethylhexyl-2-hydroxybenzoat(INCI: Ethylhexylsalicylat).

**Ein weiterer Gegenstand ist das Verfahren zur Herstellung der erfindungsgemäßen hydrophilen Organopolysiloxangelzubereitung** Dazu werden die Komponenten miteinander vermengt.

Das Herstellverfahren entspricht dabei genau oder im Wesentlichen dem Herstellverfahren für die hydrophoben Elastomergele. Die hydrophoben Elastomergele werden in der Regel in einem einstufigen Verfahren hergestellt. In Ausnahmefällen ist auch eine zweistufige Vorgehensweise bekannt, bei der zwei unterschiedliche ungesättigte Verbindungen in der Regel mit unterschiedlicher Reaktivität stufenweise mit Si-H-funktionellen Organopolysiloxanen umgesetzt werden.

Enthält das Herstellverfahren einen Schritt zur Viskositätseinstellung durch Zugabe eines Verdünnungsmittels nach beendeter Hydrosilylierungsreaktion, kann die Verbindung (3) in diesem Schritt zugesetzt werden. Ist kein solcher Schritt im Herstellverfahren des hydrophoben Organopolysiloxangels vorhanden, so erfolgt die Zugabe der Verbindung (3) in einem eigenen Dosierschritt.

Ein großer Vorteil ist somit, dass eine eigene Herstellung der hydrophilen Elastomergele in einem ein- oder vorzugsweise zweistufigen Verfahren entfällt.

Somit gewinnt man aus einem Prozess zwei deutlich unterschiedliche Produkte mit einer minimalen Anpassung im Verfahren.

Dabei ist insbesondere vorteilhaft, dass die Verbindungen (3) , wie aus dem bekannten Stand der Technik zu entnehmen ist, selbst ohnehin für andere Zwecke hergestellt und eingesetzt werden oder auch für andere Zwecke als Emulgatoren, Schaumstabilisatoren, Formulierungshilfsmittel oder dergleichen einsetzbar sind und eingesetzt werden und somit kommerziell leicht zugänglich sind.

Es ist auch bezeichnend für die Einfachheit des erfindungsgemäßen Verfahrens, dass der Eigenschaftswechsel von einem hydrophoben zu einem hydrophilen Gel mit allen praktisch relevanten und im Stand der Technik gefundenen siliconbasierenden hydrophoben Elastomergeltechniken funktioniert.

Aus einem hydrophoben Organopolysiloxangel erhält man ein erfindungsgemäßes hydrophiles Organopolysiloxangel, indem man nach der Zugabe der Stopperverbindung eine Verbindung (3) zumischt. Dies kann geschehen, indem man die Verbindung (3) zusammen mit dem Verdünnungsmittel in dem optionalen Verdünnungsschritt der Herstellung des hydrophoben Organopolysiloxangels zudosiert oder falls kein Verdünnungsschritt nach der Zugabe der Stopperverbindung vorgesehen ist, indem man die Verbindung (3) in einem eigenen Dosierschritt zusetzt.

Dabei kommen hochscherende Mischtechniken nach Stand der Technik zum Einsatz wie man sie auch bei dem optionalen Verdünnungsschritt einsetzen würde.

Das Verdünnen und Vermischen kann durch intensives Mischen und Dispergieren mit geeigneten Rühraggregaten oder in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Dabei werden Homogenisiergeräte und Verfahren gemäß Stand der Technik eingesetzt.

Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben.

Für die Herstellung ist wesentlich, dass durch die Zugabe der Verbindung (3), die selbst keine cremig-gelige Konsistenz aufweist, die Viskosität der erhaltenen erfindungsgemäßen hydrophilen Organopolysiloxangelzubereitung im Vergleich zur Herstellung ohne hydrophile Komponente niedriger ist. Ist das gleiche Viskositätsniveau gewünscht, wie beim hydrophoben Organopolysiloxan, so ist eine entsprechend geringere Menge Verdünnungsmittel (4) einzusetzen. Die jeweils erforderliche Reduzierung an Verdünnungsmittel (4) ist experimentell zu ermitteln und abhängig von der jeweiligen hydrophilen Komponente. Es führt daher nicht zum gleichen Ergebnis, wenn man beispielsweise seitens eines Formulierers die hydrophobe und die hydrophile Komponente miteinander mischt. Die resultierende Viskosität aus dieser Vorgehensweise entspricht nicht der Viskosität, des hydrophoben Organopolysiloxangels in seiner ursprünglichen Lieferform. Insbesondere wird daher bei der Erstellung von Rezepturen von kosmetischen Zubereitungen nicht das gleiche Ergebnis erhalten. Es ist bekannt, dass die Viskosität der Elastomergele einen Einfluss auf das durch sie verursachte Hautgefühl hat.

Ein wesentlicher Vorteil der erfindungsgemäßen hydrophilen Organopolysiloxangelzubereitungen ist ihre verbesserte Kompatibilität mit polaren oder hydrophilen organischen Substanzen, beispielsweise Glycerin, und sogar Wasser. Diese wichtigen Kosmetikinhaltsstoffe sind mit herkömmlichen Organopolysiloxangelen nicht, oder nur in sehr geringen Mengen mischbar. Die erfindungsgemäßen hydrophilen Organopolysiloxangelzubereitungen sind in der Lage, äußerst polare Stoffe wie Wasser oder Glycerin unter Beibehalt der viskosen Gelstruktur und Ausbildung einer einphasigen, homogenen Mischung aufzunehmen.

Durch ihre verbesserte Kompatibilität mit polaren und hydrophilen organischen Substanzen und sogar Wasser, zeigen die erfindungsgemäßen hydrophilen Organopolysiloxangelzubereitungen auch in wasser- oder alkoholbasierten Formulierungen einen verdickenden Effekt und verleihen solchen Formulierungen ein bevorzugtes seidiges Hautgefühl.

Die erfindungsgemäßen hydrophilen Organopolysiloxangelzubereitungen weisen gegenüber dem Stand der Technik nicht nur den Vorteil auf, dass der Aufwand zur Erstellung eines umfassenden Portfolios erheblich reduziert wird, sondern weisen auch technische Vorteile auf. Gegenüber hydrophilen Organopolysiloxangelen gemäß US2016311980A1 wurde gefunden, dass die hier erfindungsgegenständlichen hydrophilen Organopolysiloxangelzubereitungen bei vergleichbarer Zusammensetzung und Fähigkeit zur Wasseraufnahme transparent hergestellt werden können und ein besseres Hautgefühl aufweisen, als vergleichbare Gele gemäß US2016311980A1 . Dafür ist im experimentellen Teil ein Beispiel angegeben.

Ein transparentes Organopolysiloxangel kann dabei ein größeres Anwendungsspektrum überstreichen, denn es kann sowohl in transparente als auch in opake Formulierungen ohne Störung des Erscheinungsbildes der Endformulierung eingesetzt werden.

Die erfindungsgemäßen hydrophilen Organopolysiloxangelzubereitungen unterscheiden sich von hydrophilen Zubereitungen, die aus einem hydrophoben Gel dadurch erhalten werden, dass man eine hydrophile siliconfreie organische Komponente in ein hydrophobes Organopolysiloxangel einarbeitet. Die erfindungsgemäßen hydrophilen Organopolysiloxangelzubereitungen können transparent erhalten werden, weil sie aus Komponenten bestehen, die miteinander verträglich und mischbar sind. Das ist bei Zubereitungen aus organischen hydrophilen und hydrophoben Organopolysiloxangelen nicht der Fall. Letztere Zubereitungen sind aufgrund der Unverträglichkeit ihrer Komponenten trüb und neigen dazu sich in Gegenwart von Wasser wieder voneinander zu trennen, so dass die organische Komponente beispielsweise als Emulgator für die Siliconkomponente fungiert, jedoch deren Leistungsmerkmale in keiner Weise unterstützt oder steigert. Die erfindungsgemäßen hydrophilen Organopolysiloxangelzubereitungen zeigen dieses Verhalten, d.h. es wurde gefunden, dass die Leistungsmerkmale der hydrophoben Organopolysiloxangele durch die Beimischung der hydrophilen Komponente (3) verbessert werden konnten. Die Komponenten der erfindungsgemäßen hydrophilen Organopolysiloxangelzubereitungen bleiben dabei homogen vereint und trennen sich nicht in die einzelnen Bestandteile, die dann anschließend unabhängig voneinander eine ggf. verschiedene Funktion ausüben.

Bevorzugt wird die erfindungsgemäße hydrophile Organopolysiloxangelzubereitung unter Verwendung von standardmäßigen hochscherenden Mischtechniken bis zu einer cremigen Konsistenz homogenisiert. Hierfür geeignete Technologien sind die gleichen wie in diesem Text für den optionalen Verdünnungsschritt und die Zugabe der Verbindung (3) genannten.

Unter cremig in Bezug auf die Organopolysiloxangelzubereitung ist zu verstehen, dass das Ausgangsgel bis zu einer cremigen Konsistenz geschert worden ist. Die resultierende cremige Organopolysiloxangelzubereitung kann je nachdem gießbar oder verhältnismäßig steif sein. Durch das Attribut cremig unterscheiden sich diese gescherten Gele, die transparent oder opak sein können, von den unmittelbar durch Gelierung der reaktiven Bestandteile hergestellten Gelen.

Unter lagerstabil im Rahmen dieser Erfindung ist zu verstehen, dass sich die gebildeten hydrophilen Organopolysiloxangelzubereitungen innerhalb von 6 Monaten Lagerung bei Raumtemperatur nicht in zwei oder mehr Phasen entmischen. Bevorzugt ändert sich die Weichheit des Gels in diesem Zeitraum nicht.

Dem Fachmann ist verständlich, dass das Aufnahmevermögen für Verdünnungsmittel (4) aufgrund der dreidimensionalen Netzwerkstruktur von Organopolysiloxangelen im Allgemeinen beschränkt ist und in Abhängigkeit von der Netzwerkstruktur und Netzwerkzusammensetzung variieren kann. Ist das Aufnahmevermögen für Verdünnungsmittel(4) überschritten, so ist die Bildung einer Verdünnungsmittelphase neben einer Gelphase zu erkennen. In diesem Zusammenhang zeigt auch die Komponente (3) eine gewisse verdünnende Wirkung, da sie selbst keine hochviskose Gelstruktur besitzt. Ebenso einfach wie die Verwendung der Verdünnungsmittel (4), fügt sich der Einsatz der Komponente (3) in den bestehenden Herstellprozess der als Edukt eingesetzten hydrophoben Organopolysiloxangelen ein.

**Ein weiterer Gegenstand der Erfindung ist die Verwendung der erfindungsgemäßen hydrophilen Organopolysiloxangelzubereitung** in kosmetischen Zubereitungen oder Zubereitungen für die Körper- und Gesundheitspflege oder in Wasch- und Reinigungsprodukten oder in Produkte zur langanhaltenden Beduftung oder Insektenabwehr.

Die erfindungsgemäßen hydrophilen Organopolysiloxangelzubereitungen sind besonders bevorzugt geeignet für kosmetische Anwendungen, und werden daher bevorzugt in kosmetischen Zusammensetzungen eingesetzt. Sie eignen sich aber auch für andere Anwendungen, beispielsweise für medizinische und technische Anwendungen.

Sie sind überraschenderweise in der Lage Wasser in die Gelstruktur aufzunehmen, ohne dass die Zubereitung in ihre Bestandteile aufgespalten wird. Die physikalische Mischung verhält sich also unerwarteterweise wie eine homogene Substanz aus nur einer Spezies. Durch die Fähigkeit Wasser in die Gelstruktur aufzunehmen, wird die Verträglichkeit der erfindungsgemäßen hydrophilen Organopolysiloxangelzubereitungen mit wässrigen kosmetischen Zubereitungen verbessert und ihre Einarbeitung erleichtert. Im Gegensatz zu marktüblichen Produkten, die Zubereitungen aus Organopolysiloxangelen und organischen Emulgatoren enthalten, enthalten die erfindungsgemäßen hydrophilen Organopolysiloxangelzubereitungen nur Siliconkomponenten und können so das ästhetische Hautgefühl ohne Abstriche in der Leistungsfähigkeit mit den Vorteilen verbesserter Verträglichkeit verbinden.

Die erfindungsgemäßen hydrophilen Organopolysiloxangelzubereitungen haben besonderen Wert in Körperpflegeprodukten. Sie können sanft auf der Haut verteilt werden und können daher alleine verwendet werden oder mit anderen Körperpflegeproduktbestandteilen gemischt werden, um eine Vielzahl von Körperpflegeprodukten zu bilden.

Beispiele von Körperpflegeproduktbestandteilen sind Ester, Wachse, Öle und Fette von tierischem oder pflanzlichem Ursprung, Fettalkohole, Fettsäuren, Alkylester von Fettsäuren, Kohlenwasserstoffe und -wachse, Wasser, organische Lösungsmittel, Parfüme, oberflächenaktive Mittel, öllösliche Vitamine, wasserlösliche Vitamine, öllösliche Arzneimittel, wasserlösliche Arzneimittel, UV-Absorber und aktive pharmazeutische Verbindungen.

Insbesondere sind die erfindungsgemäßen hydrophilen Organopolysiloxangelzubereitungen in Antiperspirantien und Deoperspirantien geeignet, da sie ein trockenes Gefühl zurücklassen und die Haut nicht während der Verdampfung abkühlen. Sie sind gleitfähig und verbessern die Eigenschaften von Hautcremes, Hautpflegelotionen, Moisturizern, Gesichtsbehandlungen, wie z.B. Akne- oder Faltenentfernern, Körper- und Gesichtsreinigern, Badeölen, Parfüms, Kölnisch Wasser, Sachets, Sonnenschutzmittel, Preshave- und Aftershave-Lotionen, flüssigen Seifen, Rasierseifen und Rasierschäumen. Sie können in Haarshampoos, Haarkonditionierern, Haarsprays, Mousses, Dauerwellenmittel, Haarentfernungsmittel und Nagelhautabdeckungen verwendet werden, um Glanz und Trockengleiten zu verbessern und Konditioniervorteile bereitzustellen.

In Kosmetika fungieren sie als Verteilungsmittel für Pigmente in Make-ups, Farbkosmetika, Grundierungen, Rouge, Lippenstiften, Lippenbalsam, Lidstrich, Mascara, Ölentfernern und Farbkosmetikentfernern. Sie sind als Verabreichungssysteme für hierin beispielhaft genannte öllösliche aktive Bestandteile, wie z.B. Vitamine, Arzneimittel und UV Absorber geeignet. Wenn sie in Stiften, Gelen, Lotionen, Cremes, Roll-ons eingesetzt werden, verleihen die Elastomere ein trockenes, seidig sanftes Gefühl. In Kosmetika und anderen Hautpflegeprodukten eingebracht, verleihen die erfindungsgemäßen hydrophilen Organopolysiloxangelzubereitungen einen Mattierungseffekt. Zusätzlich zeigen die erfindungsgemäßen hydrophilen Organopolysiloxangelzubereitungen eine Vielzahl vorteilhafter Eigenschaften, wie z.B. Klarheit, Lagerstabilität und Einfachheit der Herstellung. Daher haben sie einen breiten Anwendungsbereich, insbesondere in Antiperspirantien, Deodorantien, Hautpflegeprodukten, in Parfüms als Träger und für die Haarkonditionierung, beispielsweise in Hair-Balm oder Hair-Mask Conditioner.

Die erfindungsgemäßen hydrophilen Organopolysiloxangelzubereitungen haben Verwendung über den Körperpflegebereich hinaus, einschließlich deren Verwendung als Füllstoff oder Isolierungsmaterial für elektrische Kabel, Boden- oder Wasserbarrieren für Bodenstabilisierung oder als Ersatz für Epoxymaterialen die in Bauteilen in der elektronischen Industrie verwendet werden. Sie sind ebenfalls als Träger für vernetzte Siliconkautschukteilchen geeignet. In diesen Anwendungen erlauben sie (i) die Einfachheit des Einbringens von Teilchen in solche Silicon- oder organische Phasen, wie Dichtmittel, Farben, Beschichtungen, Fette, Klebstoffe, Antischaummittel und Gießharzverbindungen, und (ii) stellen modifizierte rheologische, physikalische oder energieabsorbierende Eigenschaften solcher Phasen, entweder in ihrem reinen oder in ihrem Endzustand, bereit.

Zusätzlich sind die erfindungsgemäßen hydrophilen Organopolysiloxangelzubereitungen in der Lage, als Träger für Pharmazeutika, Biozide, Herbizide, Pestizide und andere biologische aktive Substanzen zu wirken.

Weiterhin finden die erfindungsgemäßen hydrophilen Organopolysiloxangelzubereitungen Anwendung als Additive für nichtgewebte Trägersubstrate auf Cellulosebasis oder nichtgewebte synthetische Trägersubstrate, die in feuchten Reinigungstüchern wie Feuchttüchern, feuchten Papiertüchern und feuchten Handtüchern verwendet werden, die im Allgemeinen für Körperhygiene--und Haushaltsreinigungszwecke vermarktet werden.

Die erfindungsgemäßen hydrophilen Organopolysiloxangelzubereitungen können als Träger zur gesteuerten und leicht regulierten Freisetzung einer flüchtigen aktiven organischen Substanz in die freie Atmosphäre verwendet werden, wenn sie mit dieser vermischt werden. Die flüchtige Substanz kann insbesondere ein Parfüm sein oder ein Insektizid oder ein Stoff, der Insekten abwehrt.

In dieser Verwendung finden die erfindungsgemäßen hydrophilen Organopolysiloxangelzubereitungen breite Anwendung, beispielsweise bei der Ausrüstung von Fasern, Textilien und Stoffen aus Baumwolle oder Kunstfasern, Geweben, Tüchern, auch Papiertüchern, Toilettenpapier oder Wischpapier, wie Servietten oder Küchenrolle, oder Vlies, zur langanhaltenden, kontrollierten Beduftung oder Insektenabwehr. Die Mischung aus der erfindungsgemäßen hydrophilen Organopolysiloxangelzubereitung und der flüchtigen aktiven organischen Substanz kann auch in Waschmaschinen und Wäschetrockner direkt als solche oder als Zusatz zu Waschmitteln und Weichspülern auf Stoffe und Textilien appliziert werden.

Die Verwendung der erfindungsgemäßen hydrophilen Organopolysiloxangelzubereitung als Träger zur gesteuerten und leicht regulierten Freisetzung einer flüchtigen aktiven organischen Substanz findet insbesondere Anwendung in den oben genannten kosmetischen Anwendungen, wo sie einen Zusatzeffekt zum dort beschrieben Effekt erzielen können, indem sie beispielsweise ein Parfüm gesteuert abgeben. Die erfindungsgemäßen hydrophilen Organopolysiloxangelzubereitungen können auch in Insektenabwehrpräparaten eingesetzt werden, wo sie ein Insektizid oder einen Stoff, der Insekten abwehrt, abgeben. Solche Produkte können beispielsweise direkt auf die Haut oder auf die Kleidung aufgebracht werden.

In einer weiteren Anwendung können die Mischung aus der erfindungsgemäßen hydrophilen Organopolysiloxangelzubereitung und der flüchtigen aktiven organischen Substanz zur kontrollierten Beduftung oder Insektenabwehr in geschlossenen Räumen, wie zum Beispiel in Wohnräumen, Geschäftsräumen, WCs oder Fahrzeugen, wie Busse und Autos, eingesetzt werden.

### Beispiele:

Die nachfolgenden Beispiele dienen dazu, die Erfindung weiter zu erläutern und ihre Funktion und Anwendung in der Praxis zu beschreiben. Sie sind in diesem Sinne illustrierend zu verstehen, nicht beschränkend.

In den Beispielen werden physikalische Größen angegeben, die nach den nachfolgend beschriebenen Messmethoden bestimmt wurden. Falls Angaben zur genauen Nachvollziehbarkeit eines Messwertes im Beispieltext fehlen, sind diese in den hier nachfolgenden Beschreibungen der Messmethoden bereits angegeben. D.h. in diesem Fall gelten als weitere Angaben die in den Texten zu den Messmethoden angegebenen.

Im nachfolgenden Text bedeutet Me einen Methylrest -CH₃

### Analytische Methoden:

Die Viskosität der Organopolysiloxangele/ Organopolysiloxangelzubereitung wurden nach DIN EN ISO 3219 bei Schergeschwindigkeit 1/s und 25°C bestimmt.

Die Viskosität der Organopolysiloxane, wie Si-H-haltige Vernetzer, Organopolysiloxanharze und Polydimethylsiloxane, wurden nach DIN 53019 im linearen Bereich bei 25°C bestimmt.

Die Jodzahl wurde nach DIN 53241-1 gemäß dem Verfahren nach Wijs bestimmt.

Gelpermeationschromatografie zur Bestimmung des gewichtsmittleren Molekulargewichts Mw wurde durchgeführt nach ISO 16014-1 und ISO 16014-3.

Die erfindungsgegenständlichen Organopolysiloxangelzubereitungen bewirken in kosmetischen Anwendungen sensorische Vorteile, indem sie die Verteilbarkeit des Produkts auf der Haut verbessern und dem Produkt ein seidig sanftes Gefühl verleihen. Vergleichbar sind die Organopolysiloxangele dabei in ihrer Leistungsfähigkeit nur dann, wenn sie für die sensorische Prüfung auf eine einheitliche Viskosität eingestellt werden. Als besonders vorteilhaft hat sich hierfür eine Viskosität im Bereich von 75000 - 120000 mPas bei 25°C erwiesen. Ein Kriterium für die erfolgreiche Herstellung der Organopolysiloxangele ist daher die Möglichkeit, diesen Viskositätskorridor einzustellen. Ist dies nicht möglich, ist die Vergleichbarkeit mit den anderen Organopolysiloxangelen nicht gegeben.

Die Beurteilung der sensorischen Eigenschaften der nachfolgend in den Beispielen beschriebenen Organopolysiloxangele erfolgte durch eine geschulte Gruppe von 5 Probanden (Panelisten).

Die Panelisten haben auf den gereinigten Unterarm auf einer kreisrunden Fläche von 20 cm² jeweils 0,05 g des Produktes aufgetragen und die Organopolysiloxangele im Hinblick auf ihre Verteilbarkeit relativ zueinander verglichen. Das Auftragen erfolgte mit dem Zeigefinger oder Mittelfinger und einer Rotationsgeschwindigkeit von zwei Umdrehungen pro Sekunde. Insgesamt wurden 30 Umdrehungen durchgeführt. Nach einer Wartezeit von 60 Sekunden wurden die Rückstände der Organopolysiloxangele im Hinblick auf ihre Seidigkeit relativ zueinander verglichen.

Relativ zueinander direkt vergleichbar sind dabei stets nur die Organopolysiloxangele, die unter Verwendung des gleichen oder zumindest eines gleichartigen Verdünnungsmittels (4), also insbesondere mit einem flüchtigen oder einem nicht flüchtigen, hergestellt wurden. Da durch unterschiedliche Verdünnungsmittel (4) ein unterschiedliches Verhalten beim Auftragen und beim Rückstand resultiert, müssen Produkte mit unterschiedlichen Verdünnungsmitteln (4) jeweils getrennt voneinander bewertet werden.

### Beispiel 1 (erfindungsgemäß):

Ein 2000 ml-Reaktionsgefäß aus Glas wird mit einem Kühler mit aufgesetzter Stickstoffeinleitung, Heizmantel, Ankerrührer und Temperaturregelung ausgestattet. In das Reaktionsgefäß werden 515 g eines linearen trimethylsilylterminierten Polydimethylsiloxanes mit einer Viskosität von 5 mPas bei 25°C gegeben.

Danach werden 80 g eines Si-H-haltigen Polydimethylsiloxans der Formel (Me₃SiO_{1/2})(Me₂SiO_{2/2})ₐ(MeHSiO_{2/2})_{b}(Me₃SiO_{1/2}) zugegeben, mit a:b = 55:1 und einer Kettenlänge von a+b = 134 Wiederholungseinheiten und 20 g eines Si-H-haltigen Polydimethylsiloxans (Me₃SiO_{1/2})(Me₂SiO_{2/2})ₐ(MeHSiO_{2/2})_{b}(Me₃SiO_{1/2}) mit a:b = 50:1 und einer Kettenlänge von 450 einschließlich der terminalen Einheiten zugegeben.

Dann fügt man 107,5 g einer 50%-igen Lösung eines MQ-Harzes (M / M^{Vi} / Q = 7,6 / 1 / 11,4; Mₙ = 2570, M_{w} = 5440, Jodzahl=18; M = Me₃SiO_{1/2}, M^{Vi} = Me₂ViSiO_{1/2}, Q = SiO_{4/2} mit Me = Methylrest und Vi= Vinylrest) in dem gleichen linearen trimethylsilylterminierten Polydimethylsiloxan mit einer Viskosität von 5 mPas bei 25°C, das auch als Verdünnungsmittel verwendet wird, hinzu. Zuletzt gibt man 0,7 g einer Mischung von Platin-1,3-Divinyl-1,1,3,3-tetramethyldisiloxan-Komplex in Divinyltetramethyldisiloxan hinzu, wobei die Mischung so eingestellt ist, dass die zugesetzte Menge 52 Gew.-ppm Pt entspricht, bezogen auf die Summe der Masse aus dem MQ-Harz und den beiden Si-H-haltigen Organopolysiloxanen. Das Reaktionsgefäß wird geschlossen und 5 min mit Stickstoff gespült.

Anschließend wird die Reaktionsmischung bei einer Rührgeschwindigkeit von etwa 200 U/min auf 95°C erhitzt, wobei eine Heizrate von 45°C/h angewendet wird. Die Gelbildung erfolgt innerhalb von 30 Minuten nachdem die Innentemperatur von 95°C erreicht ist. Nach erfolgter Gelbildung wird die Heizung abgeschaltet und weitere 60 Minuten gerührt.

Danach werden 10,0 g Polydimethylsiloxan mit 3-Mercaptopropylgruppen und einer Viskosität von 190 mPas bei 25°C und einem Mercaptangehalt (SH-Gehalt) von 0,29 Gew.-% als Stopper hinzugegeben.

An dieser Stelle wird der Ansatz in zwei gleich große Portionen aufgeteilt, die separat weiterbearbeitet werden. Ein Teil wird ohne Zusatz eines Glucosidreste enthaltenden Polyorganosiloxans (3a) bearbeitet, der zweite Teil wird unter Verwendung eines Glucosidreste enthaltenden Polyorganosiloxans (3a) aufgearbeitet.

Man fügt zur ersten Hälfte **43,2 g des als Verdünnungsmittel** (4) eingesetzten Polydimethylsiloxans mit einer Viskosität von 5 mPas bei 25°C zu und rührt es 5 Minuten lang mit einem ULTRA-TURRAX^{®} T 50 bei 6000 U/min ein. **Man wiederholt diesen Vorgang noch zweimal mit jeweils der gleichen Menge Verdünnungsmittel (4).** Auf diesem Wege erhält man ein cremiges, transparentes Gel mit sehr glatter Konsistenz, das zur Verwendung in Kosmetikprodukten geeignet ist.

Der Festgehalt, also der gesamte Gehalt an Netzwerk aus MQ-Harz und den beiden Si-H-haltigen Organopolysiloxanen, sowie dem Stopper und dem Katalysator im Verdünnungsmittel (4), beträgt nach Verdünnen **16,4 Gew.-%.**

Das erhaltene Organopolysiloxangel besitzt eine Viskosität von **107 000 mPas bei 25°C.**

Man fügt zur zweiten Hälfte **42,3 g des als Verdünnungsmittel** (4) eingesetzten Polydimethylsiloxans mit einer Viskosität von 5 mPas bei 25°C und rührt es 5 Minuten lang mit einem ULTRA-TURRAX^{®} T 50 bei 6000 U/min ein. Anschließend werden **25 g** des Glucosidreste enthaltenden Organopolysiloxans (3a) gemäß Beispiel 14

zugegeben und weitere 5 Minuten lang mit einem ULTRA-TURRAX^{®} T 50 bei 6000 U/min eingerührt.

Auf diesem Wege erhält man ein cremiges, transparentes Gel mit sehr glatter Konsistenz, das zur Verwendung in Kosmetikprodukten geeignet ist.

Der Festgehalt, also der gesamte Gehalt an Netzwerk aus MQ-Harz und den beiden Si-H-haltigen Organopolysiloxanen, sowie dem Stopper und dem Katalysator im Verdünnungsmittel (4), beträgt nach Verdünnen **20,5 Gew.-%.**

Das erhaltene Organopolysiloxangel besitzt eine Viskosität von **105 000 mPas bei 25°C.**

Nach der Bewertung der Panelisten sind beide so erhaltene Organopolysiloxangel sehr gut verteilbar. Die Rückstände wurden als reichhaltig und mehrheitlich als überwiegend seidig und samtig eingestuft.

Das aus der ersten Hälfte des Ansatzes ohne Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **40** g Wasser in das erhaltene Gel einrühren bis Sättigung eintritt. Eine gesättigte Mischung ist daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus der zweiten Hälfte des Ansatzes unter Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **1200** g Wasser in das erhaltene Gel einrühren bis Sättigung eintritt, die in der gleichen Weise erkennbar ist, wie oben beschrieben.

Nach der Bewertung der Panelisten ist das erhaltene Organopolysiloxangel aus dem zweiten Teil des Ansatzes unter Verwendung des Glucosidreste enthaltenden Polyorganosiloxans (3a) sehr gut verteilbar. Der Rückstand wurde als reichhaltig und mehrheitlich als überwiegend seidig und samtig eingestuft.

### Beispiel 2 (erfindungsgemäß):

Ein 2000 ml-Reaktionsgefäß aus Glas wird mit einem Kühler mit aufgesetzter Stickstoffeinleitung, Heizmantel, Ankerrührer und Temperaturregelung ausgestattet. Das Experiment wird bei einer Raumtemperatur von 23°C ausgeführt. Alle Einsatzstoffe besitzen diese Temperatur.

In das Reaktionsgefäß werden 636 g eines linearen trimethylsilylterminierten Polydimethylsiloxanes mit einer Viskosität von 2 mPas bei 25°C gegeben.

Danach werden 18,6 g eines Si-H-haltigen Polydimethylsiloxans der Formel (Me₃SiO_{1/2})(Me₂SiO_{2/2})ₐ(MeHSiO_{2/2})_{b}(Me₃SiO_{1/2}) zugegeben, mit a:b = 15:1 und einer Kettenlänge von a+b = 225 Wiederholungseinheiten.

Dann fügt man 100,7 g eines vinyldimethylsiloxyterminierten Polydimethylsiloxans der Formel ((H₂C=CH)Me₂SiO_{1/2})(Me₂SiO_{2/2})ₐ((H₂C=CH)Me₂SiO_{1/2}) mit einer Viskosität von 300 mm² bei 25°C und einer Jodzahl von **4** hinzu. Zuletzt gibt man 0,08 g einer Mischung von Platin-1,3-Divinyl-1,1,3,3-tetramethyldisiloxan-Komplex in Divinyltetramethyldisiloxan hinzu, wobei die Mischung so eingestellt ist, dass die zugesetzte Menge 5,4 Gew.-ppm Pt entspricht, bezogen auf die Summe der Masse aus dem vinyldimethylsiloxyterminierten Polydimethylsiloxan und dem Si-H-haltigen Organopolysiloxan. Das Reaktionsgefäß wird geschlossen und 5 min mit Stickstoff gespült.

Anschließend wird die Reaktionsmischung bei einer Rührgeschwindigkeit von etwa 200 U/min bei Raumtemperatur gerührt. Die Gelbildung beginnt visuell gut erkennbar nach 45 Minuten. Nach einsetzender Gelbildung werden weitere 60 Minuten bei Raumtemperatur gerührt. Durch die Exothermie der Reaktion und des Wärmeeintrages durch das Rühren wird eine Endtemperatur von 38°C erhalten.

Danach werden 4,15 g Polydimethylsiloxan mit 3-Mercaptopropylgruppen und einer Viskosität von 190 mPas bei 25°C und einem Mercaptangehalt (SH-Gehalt) von 0,29 Gew.-% als Stopper hinzugegeben.

An dieser Stelle wird weiterhin verfahren wie in Beispiel 1 beschrieben. D.h. der Ansatz wird in zwei gleich große Teile geteilt, die getrennt voneinander weiterbearbeitet werden.

Zum ersten Teil fügt man **40,1 g des als Verdünnungsmittel** (4) eingesetzten Polydimethylsiloxans mit einer Viskosität von 2 mPas bei 25°C zu und rührt es 5 Minuten lang mit einem ULTRA-TURRAX^{®} T 50 bei 6000 U/min ein. **Man wiederholt diesen Vorgang noch zweimal mit der jeweils gleichen Menge Verdünnungsmittel (4) von 40,1 g.** Es wird dabei kein weiterer Stopper hinzugefügt. Auf diesem Wege erhält man ein cremiges, transparentes Gel mit sehr glatter Konsistenz, das zur Verwendung in Kosmetikprodukten geeignet ist.

Der Festgehalt, also der gesamte Gehalt an Netzwerk aus vinylterminiertem Polysiloxan und den beiden Si-H-haltigen Organopolysiloxanen, sowie dem Stopper und dem Katalysator im Verdünnungsmittel (4), beträgt nach Verdünnen **12,4 Gew.-%.** Das erhaltene Organopolysiloxangel besitzt eine Viskosität von **97 000 mPas bei 25°C.**

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

Zum zweiten Teil fügt man **39,6 g des als Verdünnungsmittels (4)** eingesetzten Polydimethylsiloxans mit einer Viskosität von 2 mPas bei 25°C zu, sowie **25,0 g** des Glucosidreste enthaltenden Organopolysiloxans (3a) gemäß Beispiel 14 und rührt diese 5 Minuten lang mit einem ULTRA-TURRAX^{®} T 50 bei 6000 U/min ein. **Man wiederholt diesen Vorgang noch zweimal mit der jeweils gleichen Menge Verdünnungsmittel (4) von 39,6 g.** Es wird dabei kein weiterer Stopper oder Glucosidrest enthaltendes Polyorganosiloxan mehr hinzugefügt. Auf diesem Wege erhält man ein cremiges, transparentes Gel mit sehr glatter Konsistenz, das zur Verwendung in Kosmetikprodukten geeignet ist.

Der Festgehalt, also der gesamte Gehalt an Netzwerk aus vinylterminiertem Polysiloxan und den beiden Si-H-haltigen Organopolysiloxanen, sowie dem Stopper und dem Katalysator im Verdünnungsmittel (4), beträgt nach Verdünnen **13,5 Gew.-%.** Das erhaltene Organopolysiloxangel besitzt eine Viskosität von **94 000 mPas bei 25°C.**

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

Das aus der ersten Hälfte des Ansatzes ohne Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **45** g Wasser in das erhaltene Gel einrühren bis Sättigung eintritt. Eine gesättigte Mischung daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus der zweiten Hälfte des Ansatzes unter Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **1200** g Wasser in das erhaltene Gel einrühren bis Sättigung eintritt, die in der gleichen Weise erkennbar ist, wie oben beschrieben.

Nach der Bewertung der Panelisten ist das erhaltene Organopolysiloxangel aus dem zweiten Teil des Ansatzes unter Verwendung des Glucosidreste enthaltenden Polyorganosiloxans (3a) sehr gut verteilbar. Der Rückstand wurde als reichhaltig und mehrheitlich als überwiegend seidig und samtig eingestuft.

### Beispiel 3 (erfindungsgemäß):

Die Vorgehensweise entspricht im Wesentlichen der in Beispiel 2 beschriebenen. Im Unterschied zu Beispiel 2 wird als Verdünnungsmittel (4) anstelle eines flüchtigen trimethylsilylterminierten linearen Polydimethylsiloxans mit einer Viskosität von 2 mPas bei 25°C ein nicht flüchtiges trimethylsilylterminiertes lineares Polydimethylsiloxan mit einer Viskosität von 5 mPas bei 25°C eingesetzt. Die ins Reaktionsgefäß vorgelegte Menge an Verdünnungsmittel (4) beträgt 780,0 g.

Von dem Si-H-haltigen Polydimethylsiloxan, das auch in Beispiel 2 verwendet wurde, werden hier 40,0 g eingewogen. Zusätzlich wird ein Si-H-haltiges Polydimethylsiloxan der Formel (Me₃SiO_{1/2})(Me₂SiO_{2/2})ₐ(MeHSiO_{2/2})_{b}(Me₃SiO_{1/2}) mit a:b = 15: 1 und einer Kettenlänge von 75 Wiederholungseinheiten einschließlich der terminalen Gruppen in einer Menge von 156,0 g eingewogen. Statt des vinylterminierten Polydimethylsiloxans aus Beispiel 2 werden 6,0 g Hexadien verwendet. Zuletzt werden 0,04 g einer Mischung von Platin-1,3-Divinyl-1,1,3,3-tetramethyldisiloxan-Komplex in Divinyltetramethyldisiloxan zugegeben, wobei die Mischung so eingestellt ist, dass die zugesetzte Menge 20 Gew.-ppm Pt entspricht, bezogen auf die Summe der Masse aus dem Hexadien und den beiden Si-H-haltigen Polydimethylsiloxanen.

Zum Stoppern werden 14,0 g Polysiloxan mit 3-Mercaptopropylgruppen und einer Viskosität von 190 mPas bei 25 °C und einem Mercaptangehalt (SH-Gehalt) von 0,29 Gew.-% als Stopper hinzugegeben.

Es wird 5 Minuten lang mit einem ULTRA-TURRAX^{®} T 50 bei 6000 U/min gerührt und man erhält ein cremiges, transparentes Gel mit sehr glatter Konsistenz, das zur Verwendung in Kosmetikprodukten geeignet ist.

Der finale Festgehalt, d.h. der Gehalt an Netzwerk aus dem Si-H-haltigen Polydimethylsiloxanen und dem Hexadien zusammen mit der Menge eingesetztem Katalysator und dem Stopperöl liegt bei **21,6** Gew.-%. Die Viskosität des erhaltenen Organopolysiloxangels beträgt **360 000 mPas.**

An dieser Stelle wird im Prinzip weiterhin verfahren wie in Beispiel 1 beschrieben. D.h. der Ansatz wird in zwei gleich große Teile geteilt, die getrennt voneinander weiterbearbeitet werden.

Nach der Bewertung der Panelisten ist das erhaltene Organopolysiloxangel sehr gut verteilbar. Der Rückstand wurde als reichhaltig und mehrheitlich als trocken, seidig und samtig eingestuft.

Zum zweiten Teil fügt man **25,0 g** des Glucosidreste enthaltenden Organopolysiloxans (3a) gemäß Beispiel 14 und rührt den Stopper und das Polyglucosidreste enthaltende Polyorganosiloxan (3b) 5 Minuten lang mit einem ULTRA-TURRAX^{®} T 50 bei 6000 U/min ein. Auf diesem Wege erhält man ein cremiges, transparentes Gel mit sehr glatter Konsistenz, das zur Verwendung in Kosmetikprodukten geeignet ist.

Der Festgehalt, also der gesamte Gehalt an Netzwerk aus Hexadien und den beiden Si-H-haltigen Organopolysiloxanen, sowie dem Stopper und dem Katalysator im Verdünnungsmittel (4), beträgt nach Verdünnen **22,2 Gew.-%.**

Das erhaltene Organopolysiloxangel besitzt eine Viskosität von **240 000 mPas bei 25°C.**

Nach der Bewertung der Panelisten ist das erhaltene Organopolysiloxangel sehr gut verteilbar. Der Rückstand wurde als reichhaltig und mehrheitlich als überwiegend seidig und samtig eingestuft.

Das aus der ersten Hälfte des Ansatzes ohne Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **45** g Wasser in das erhaltene Gel einrühren bis Sättigung eintritt. Eine gesättigte Mischung daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus der zweiten Hälfte des Ansatzes unter Verwendung des Glucosidreste enthaltenden Polyorganosiloxans (3a) wird mit Wasser versetzt und das Wasser unter Verwendung eines ULTRA-TURRAX^{®} T 50 bei 6000 U/min eingerührt. Man kann auf diese Weise **635** g Wasser in das erhaltene Gel einrühren bis Sättigung eintritt, die in der gleichen Weise erkennbar ist, wie oben beschrieben.

Nach der Bewertung der Panelisten ist das erhaltene Organopolysiloxangel aus dem zweiten Teil des Ansatzes unter Verwendung des Glucosidreste enthaltenden Polyorganosiloxans (3a) sehr gut verteilbar. Der Rückstand wurde als reichhaltig und mehrheitlich als überwiegend seidig und samtig eingestuft.

### Beispiel 4: (Vergleichsbeispiel nicht erfindungsgemäß)

Identisches Nacharbeiten von Beispiel 8 aus US2016311980A1, wobei das Verfahren B verwendet wird.

### Beispiel 5: (erfindungsgemäß) Herstellung eines hydrophoben

Gels analog der Zusammensetzung zu Beispiel 4 und Zugabe des Polyglucosidreste enthaltenden Polyorganosiloxans (3b) gemäß Beispiel 14 in den Homogenisierschritt.

Die Vorgehensweise zur Herstellung eines hydrophoben Gels erfolgt analog zu Beispiel 1. Das heißt im Unterschied zu Beispiel 4 wird kein Hydrosilylierungsschritt einer hydrophilen Komponente ausgeführt, sondern es wird eine Hydrosilylierung nur der hydrophoben Komponenten vorgenommen, also der beiden H-Siloxanäquilibrate und des MQ-Harzes in dem durch Beispiel 4 bzw. Beispiel 8 aus = US2016311980A1 vorgegebenen Verdünnungsmittel. Dabei wird die Menge an (2-Allyloxy-ethoxy)-glucosid gemäß US 5,831,080 Beispiel 1 (A) (Spalte 8, Zeilen 23 - 43) das eine Jodzahl von 29,2 besitzt durch die Jodäquivalente Menge an MQ Harz ersetzt, wobei das MQ-Harz eine Jodzahl von 18 besitzt und das gleiche MQ-Harz ist, wie es bereits für Beispiel 1 eingesetzt wurde. Dabei ist zu beachten, dass die in Beispiel 8 gemäß US2016311980A1 angegebene Menge (2-Allyloxy-ethoxy)-glucosid nur 50% (2-Allyloxy-ethoxy)-glucosid ist und 50% 1,2-Propandiol.

Jodäquivalent bedeutet dabei, dass die Menge an MQ-Harz als Ersatz für das (2-Allyloxy-ethoxy)-glucosid so gewählt wird, dass die Menge an Vinylgruppen zur Hydrosilylierung die gleiche ist, wie für Beispiel 4. Die Menge an (2-Allyloxy-ethoxy)-glucosid wird also durch die [(29,2:2):18]-fache Menge an MQ-Harz ersetzt.

Außerdem wird abweichend von der Vorgehensweise in Beispiel 1 eine Heizrate von 90°C/Stunde gewählt, da aus der Lehre von WO2018228657A bekannt ist, dass sonst kein hochviskoses Gel aus den gewählten H-Siloxanäquilibraten gemäß Beispiel 4 erhalten werden kann. In Beispiel 4 bzw. Beispiel 8 aus US2016311980A1 wird dies kompensiert, indem man das MQ-Harz bei 95°C, also der höchsten Reaktionstemperatur zusetzt, was einer theoretischen unendlich hohen Heizrate entspricht.

Im Unterschied zu Beispiel 1 werden die beiden Si-H-haltigen Polyorganosiloxane im gleichen Verhältnis und in der gleichen Menge eingesetzt wie sie für Beispiel 4 verwendet wurden.

Auch der Katalysator und der Stopper werden nach Art und Menge gemäß Beispiel 4 gewählt, wobei der Katalysator nicht in zwei Portionen gemäß Beispiel 4 zugegeben wird, sondern wie es der Vorgehensweise von Beispiel 1 entspricht auf einmal.

Das Verdünnungsmittel (4) wird nach Art entsprechend der Vorgehensweise gemäß Beispiel 4 gewählt, allerdings um 10 Gewichtsprozent weniger davon eingesetzt.

Zusammen mit dem Stopper werden 25,0 g des Polyglucosidreste enthaltenden Polyorganosiloxans (3b) gemäß Beispiel 14 zugesetzt und zusammen mit dem Stopper mit einem Turrax gemäß Vorgehensweise nach Beispiel 4 eingearbeitet.

Die beiden Organopolysiloxangele gemäß Beispiel 4 und Beispiel 5 werden miteinander hinsichtlich ihrer kosmetisch relevanten Eigenschaften und ihres Erscheinungsbildes verglichen.

Das Organopolysiloxangel gemäß Beispiel 4 ist ein cremiges, standfestes lagerstabiles transluzentes Elastomergel mit einer Viskosität von 102 000 mPas. D.h. es ist nicht klar.

Das Organopolysiloxangel gemäß Beispiel 5 ist ein transparentes Organopolysiloxangel mit einer Viskosität von 110 000 mPas.

**Die nachfolgende Tabelle zeigt das Verhältnis Gel/Wasser, das bis zur Sättigung aufgenommen werden kann:**

| | **Beispiel 4** | **Beispiel 1 mit (3a)** |
|---|---|---|
| **Verhältnis Gel:Wasser** | **1 : 0,7** | **1: 2,45** |

Das aus Beispiel 4 erhaltene Organopolysiloxanel wird mit Wasser versetzt und das Wasser unter Verwendung eines ULTRA-TURRAX^{®} T 50 bei 6000 U/min eingerührt. Man kann auf diese Weise **350** g Wasser in das erhaltene Gel einrühren bis Sättigung eintritt. Eine gesättigte Mischung daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus Beispiel 5 erhaltene Organopolysiloxangel wird mit Wasser versetzt und das Wasser unter Verwendung eines ULTRA-TURRAX^{®} T 50 bei 6000 U/min eingerührt. Man kann auf diese Weise **1225** g Wasser in das erhaltene Gel einrühren bis Sättigung eintritt, die in der gleichen Weise erkennbar ist, wie oben beschrieben.

Nach der Bewertung der Panelisten sind die Organopolysiloxangele gemäß Beispiel 4 und Beispiel 5 beide für die Verwendung in Kosmetikprodukten sehr gut geeignet. Sie sind sehr gut verteilbar. Der Rückstand wurde jeweils als reichhaltig und mehrheitlich als überwiegend seidig und samtig eingestuft, wobei das Organopolysiloxangel gemäß Beispiel 5 Vorteile hinsichtlich der Verteilbarkeit und der Reichhaltigkeit zeigt. Auch wurde das Organopolysiloxangel gemäß Beispiel 5 als etwas samtiger eingestuft.

### Beispiel 6: (Vergleichsbeispiel nicht erfindungsgemäß):

Identisches Nacharbeiten von Beispiel 1 aus US20160317427A1 wobei das Verfahren B verwendet wird.

### Beispiel 7: (erfindungsgemäß: Herstellung eines hydrophoben Gels gemäß Beispiel 6 und während des Homogenisieren Zugabe eines Polyglycolreste umfassenden Polyorganosiloxans)

Die Vorgehensweise zur Herstellung eines hydrophoben Gels erfolgt analog zu Beispiel 1. Das heißt im Unterschied zu Beispiel 6 wird kein Hydrosilylierungsschritt einer hydrophilen Komponente ausgeführt, sondern es wird eine Hydrosilylierung nur der hydrophoben Komponenten vorgenommen, also der beiden H-Siloxanäquilibrate und des MQ-Harzes in dem durch Beispiel 6 bzw. Beispiel 1 aus US20160317427A1 vorgegebenen Verdünnungsmittel. Dabei wird die Menge an polyoxyethyliertem Allylalkohol der eine Jodzahl von ca. 52,5 besitzt durch die Jodäquivalente Menge an MQ Harz ersetzt, wobei das MQ-Harz eine Jodzahl von 18 besitzt und das gleiche MQ-Harz ist, wie es bereits für Beispiel 1 eingesetzt wurde. Jodäquivalent bedeutet dabei, dass die Menge an MQ-Harz als Ersatz für den polyoxyethylierten Allylalkohol so gewählt wird, dass die Menge an Vinylgruppen zur Hydrosilylierung die gleiche ist, wie für Beispiel 6. Die Menge an polyoxyethylierten Allylalkohol wird also durch die (52,5:18)-fache Menge an MQ-Harz ersetzt. Außerdem wird abweichend von der Vorgehensweise in Beispiel 1 eine Heizrate von 90°C/Stunde gewählt, da aus der Lehre von WO2018228657A bekannt ist, dass sonst kein hochviskoses Gel aus den gewählten H-Siloxanäquilibraten gemäß Beispiel 6 erhalten werden kann. In Beispiel 6 bzw. Beispiel 1 aus US20160317427A1 wird dies kompensiert, indem man das MQ-Harz bei 95°C, also der höchsten Reaktionstemperatur zusetzt, was einer theoretischen unendlich hohen Heizrate entspricht.

Im Unterschied zu Beispiel 1 werden die beiden Si-H-haltigen Polyorganosiloxane im gleichen Verhältnis und in der gleichen Menge eingesetzt wie sie für Beispiel 6 verwendet wurden.

Auch der Katalysator und der Stopper werden nach Art und Menge gemäß Beispiel 6 gewählt, wobei der Katalysator nicht in zwei Portionen gemäß Beispiel 6 zugegeben wird, sondern wie es der Vorgehensweise von Beispiel 1 entspricht auf einmal.

Das Verdünnungsmittel (4) wird nach Art entsprechend der Vorgehensweise gemäß Beispiel 6 gewählt, allerdings um 10 Gewichtsprozent weniger davon eingesetzt.

Zusammen mit dem Stopper werden 25,0 g des Polyoxyethylenreste enthaltenden Polyorganosiloxans gemäß Beispiel 15 zugesetzt und zusammen mit dem Stopper mit einem Turrax gemäß Vorgehensweise nach Beispiel 6 eingearbeitet.

Es ist an dieser Stelle zu bemerken, dass die hydrophoben Gele vor Zugabe des Polyglucosidreste enthaltenden

Polyorganosiloxans (3a) gemäß Beispiel 14 bzw. des polyoxyethylierte Reste tragenden Polyorganosiloxans gemäß Beispiel 15 das gleiche ist. Man könnte also bei erfindungsgemäßer Vorgehensweise sowohl die beiden hydrophilen Gele verwenden als auch das hydrophobe Basisgel, hätte also 3 statt nur 2 verkaufsfähiger Produkte. Zudem kann man dem hydrophoben Basisgel je nach Auswahl der gewählten Beimischungskomponente unterschiedliche Leistungsmerkmale verleihen.

Die beiden Organopolysiloxangele gemäß Beispiel 6 und Beispiel 7 werden miteinander hinsichtlich ihrer kosmetisch relevanten Eigenschaften und ihres Erscheinungsbildes verglichen.

Das Organopolysiloxangel gemäß Beispiel 6 ist ein cremiges, standfestes lagerstabiles transparentes Elastomergel mit einer Viskosität von 142 000 mPas.

Das Organopolysiloxangel gemäß Beispiel 7 ist ein transparentes Organopolysiloxangel mit einer Viskosität von 131 000 mPas.

**Die nachfolgende Tabelle zeigt das Verhältnis Gel/Wasser, das bis zur Sättigung aufgenommen werden kann:**

| | Beispiel 1 mit (3a) | Beispiel 6 | Beispiel 7 |
|---|---|---|---|
| Gel :Wasser | 1 : 2,45 | 1 : <0,1 | 1 : <0,1 |

Das aus Beispiel 6 erhaltene Organopolysiloxangel wird mit Wasser versetzt und das Wasser unter Verwendung eines ULTRA-TURRAX^{®} T 50 bei 6000 U/min eingerührt. Man kann auf diese Weise **40** g Wasser in das erhaltene Gel einrühren bis Sättigung eintritt. Eine gesättigte Mischung daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus Beispiel 7 erhaltene Organopolysiloxangel wird mit Wasser versetzt und das Wasser unter Verwendung eines ULTRA-TURRAX^{®} T 50 bei 6000 U/min eingerührt. Man kann auf diese Weise 45 g Wasser in das erhaltene Gel einrühren bis Sättigung eintritt, die in der gleichen Weise erkennbar ist, wie oben beschrieben.

Nach der Bewertung der Panelisten sind die Organopolysiloxangele gemäß Beispiel 6 und Beispiel 7 beide für die Verwendung in Kosmetikprodukten sehr gut geeignet. Sie sind sehr gut verteilbar. Der Rückstand wurde jeweils als reichhaltig und mehrheitlich als überwiegend seidig und samtig eingestuft, wobei alle diese Eigenschaften bei dem Organopolysiloxangel gemäß Beispiel 7 ausgeprägter sind. Insbesondere hinsichtlich der Verteilung und Reichhaltigkeit besitzt das Organopolysiloxangel gemäß Beispiel 7 deutliche Vorteile gegenüber dem Organopolysiloxangel gemäß Beispiel 6.

### Beispiel 8: (Vergleichsbeispiel, nicht erfindungsgemäß: Herstellung eines cyclodextrinbasierenden hydrophilen Elastomergels)

Identisches Nacharbeiten von Beispiel 1 aus WO2020139403A1.

### Beispiel 9: (erfindungsgemäß: Herstellung eines hydrophoben Gels gemäß Beispiel 6 und beim Homogenisieren Zugabe eines Cyclodextrinreste umfassenden Polyorganosiloxans (3c))

Die Vorgehensweise zur Herstellung eines hydrophoben Gels erfolgt analog zu Beispiel 1. Das heißt im Unterschied zu Beispiel 8 wird kein Hydrosilylierungsschritt einer hydrophilen Komponente ausgeführt, sondern es wird eine Hydrosilylierung nur der hydrophoben Komponenten vorgenommen, also des H-Siloxanäquilibrats und des MQ-Harzes in dem durch Beispiel 8 bzw. Beispiel 1 aus WO2020139403A1 vorgegebenen Verdünnungsmittel (4). Dabei wird die Menge an Allylcyclodextrin, welches 2,66 mol-% Allylgruppen besitzt durch die olefinäquivalente Menge an MQ Harz ersetzt, wobei das MQ-Harz 5 mol-% Vinylgruppen enthält. Dabei ist zu beachten, dass das Allylcyclodextrin gemäß Beispiel 1 nach WO2020139403A1 bzw. Beispiel der vorliegenden Erfindung als 50%-ige Lösung in Isopropanol zugegeben wird. Olefinäquivalent bedeutet dabei, dass die Menge an MQ-Harz als Ersatz für das Allylcyclodextrin gemäß Beispiel 1 aus WO2020139403A1 so gewählt wird, dass die Menge an Vinylgruppen zur Hydrosilylierung die gleiche ist, wie die Anzahl an Allylgruppen für Beispiel 1 nach WO2020139403A1 bzw. Beispiel 8 dieser Erfindung. Die Menge an Allylcyclodextrin wird also durch die [(2,66:2):5]-fache Menge an MQ-Harz ersetzt.

Außerdem wird abweichend von der Vorgehensweise in Beispiel 1 der vorliegenden Erfindung eine Heizrate von 90°C/Stunde gewählt, da aus der Lehre von WO2018228657A bekannt ist, dass sonst kein hochviskoses Gel aus dem gewählten H-Siloxanäquilibrat gemäß Beispiel 8 der vorliegenden Erfindung, bzw. Beispiel 1 gemäß WO2020139403A1 erhalten werden kann. In Beispiel 8 der vorliegenden Erfindung bzw. Beispiel 1 aus WO2020139403A1 wird dies kompensiert, indem man das MQ-Harz bei 80°C, also der höchsten Reaktionstemperatur zusetzt, was einer theoretischen unendlich hohen Heizrate entspricht.

Im Unterschied zu Beispiel 1 der vorliegenden Erfindung wird das Si-H-haltige Polyorganosiloxan sowie das MQ-Harz im gleichen Verhältnis und in der gleichen Menge eingesetzt wie sie für Beispiel 8 der vorliegenden Erfindung bzw. Beispiel 1 gemäß WO2020139403A1 verwendet wurden.

Auch der Katalysator, das Verdünnungsmittel (4) und der Stopper werden nach Art und Menge gemäß Beispiel 8 der vorliegenden Erfindung gewählt, wobei der Katalysator nicht in zwei Portionen gemäß Beispiel 8 zugegeben wird, sondern wie es der Vorgehensweise von Beispiel 1 der vorliegenden Erfindung entspricht auf einmal.

Zusammen mit dem Stopper werden **7,5 g** des Cyclodextrinreste enthaltenden Polyorganosiloxans (3c) gemäß Beispiel 16 zugesetzt und zusammen mit dem Stopper mit einem Turrax gemäß Vorgehensweise nach Beispiel 8 eingearbeitet.

Die beiden Organopolysiloxangele gemäß Beispiel 8 und Beispiel 9 werden miteinander hinsichtlich ihrer kosmetisch relevanten Eigenschaften und ihres Erscheinungsbildes verglichen.

Das Organopolysiloxangel gemäß Beispiel 8 ist ein cremiges, standfestes lagerstabiles transluzentes, d.h. nicht transparentes Elastomergel mit einer Viskosität von 63 000 mPas.

Das Organopolysiloxangel gemäß Beispiel 9 ist ein transparentes Organopolysiloxangel mit einer Viskosität von 72 000 mPas.

Das aus Beispiel 8 erhaltene Organopolysiloxangel wird mit Wasser versetzt und das Wasser unter Verwendung eines ULTRA-TURRAX^{®} T 50 bei 6000 U/min eingerührt. Man kann auf diese Weise **250** g Wasser in das erhaltene Gel einrühren bis Sättigung eintritt. Eine gesättigte Mischung daran zu erkennen, dass an der Oberfläche einzelne, feine Tröpfchen sitzen. Das ist ein Anzeichen dafür, dass das Netzwerk mit Lösemittel gesättigt ist. Das Gel kann jetzt kein zusätzliches Wasser mehr aufnehmen.

Das aus Beispiel 9 erhaltene Organopolysiloxangel wird mit Wasser versetzt und das Wasser unter Verwendung eines ULTRA-TURRAXO T 50 bei 6000 U/min eingerührt. Man kann auf diese Weise **300** g Wasser in das erhaltene Gel einrühren bis Sättigung eintritt, die in der gleichen Weise erkennbar ist, wie oben beschrieben.

**Die nachfolgende Tabelle zeigt das Verhältnis Gel/Wasser, das bis zur Sättigung aufgenommen werden kann:**

| | Beispiel 1 mit (3a) | Beispiel 8 | Beispiel 9 |
|---|---|---|---|
| Gel:Wasser | 1 : 2,45 | 1 : 0,5 | 1 : 0, 6 |

Nach der Bewertung der Panelisten sind die Organopolysiloxangele gemäß Beispiel 8 und Beispiel 9 beide für die Verwendung in Kosmetikprodukten sehr gut geeignet. Sie sind sehr gut verteilbar. Der Rückstand wurde jeweils als reichhaltig und mehrheitlich als überwiegend seidig und samtig eingestuft, wobei das Organopolysiloxangel gemäß Beispiel 9 Vorteile hinsichtlich der Verteilbarkeit und der Reichhaltigkeit zeigt. Auch wurde das Organopolysiloxangel gemäß Beispiel 9 als etwas samtiger eingestuft.

### Beispiel 10: (erfindungsgemäß)

Es wird ein hydrophobes Organopolysiloxangel gemäß der Vorgehensweise in Beispiel 5 erzeugt.

Abweichend zur Vorgehensweise in Beispiel 5 wird in diesem Beispiel zusammen mit dem Stopper kein Polyglucosidreste enthaltendes Polyorganosiloxan (3b) zugesetzt, sondern das Cyclodextrinreste enthaltende Polyorganosiloxan (3c) gemäß Beispiel 16.

Das erhaltene Organopolysiloxangel wird mit Wasser versetzt und das Wasser unter Verwendung eines ULTRA-TURRAX^{®} T 50 bei 6000 U/min eingerührt. Man kann auf diese Weise **1400 g** Wasser in das erhaltene Gel einrühren bis Sättigung eintritt, die in der gleichen Weise erkennbar ist, wie oben beschrieben.

Nach der Bewertung der Panelisten ist das erhaltene Organopolysiloxangel in seinen für kosmetische Anwendungen relevanten Eigenschaften mit dem unter Verwendung des Glucosidreste enthaltenden Polyorganosiloxans (3a) gemäß Beispiel 5 erhaltenen Gel gut vergleichbar. Es ist sehr gut verteilbar. Der Rückstand wurde als reichhaltig und mehrheitlich als überwiegend seidig und samtig eingestuft.

Im Unterschied zu dem Organopolysiloxangel gemäß Beispiel 5 ist es leichter verteilbar und der Rückstand ist etwas reichhaltiger.

Es ist an dieser Stelle zu bemerken, dass das hydrophobe Gel dieses Beispiels das gleiche ist wie in Beispiel 5 und Beispiel 7. Man kann also aus nur einem hydrophoben Basisgel eine Variationsbreite von hydrophilen Gelen herstellen, wobei das das hydrophobe Basisgel selbst bereits gute kosmetische Eigenschaften besitzt und selbst als Verkaufsprodukt verwendbar ist. Es ist außerdem anzumerken, dass die Menge an hydrophilen Zusätzen vergleichsweise gering ist, so dass die Menge einer Produktionscharge eine entsprechend große Menge an hydrohilen Organopolysiloxangelzubereitungen ergibt. Bei geringer Belastung der Produktionsanlage, die für die Bereitstellung der hydrophilen Polyorganosiloxane dient, wird also eine entsprechend große Menge an unterschiedlichen Organopolysiloxangelen erhalten, die alle auf der gleichen chemischen Plattform für das Organopolysiloxangel basieren. Dadurch spart sich die das Organopolysiloxangel erzeugende Produktionsstätte die Logistik und die Lagerräumlichkeiten für die Vorhaltung einer großen Bandbreite an unterschiedlichen Rohstoffen. Die Anzahl der Dosiervorrichtungen und die Anzahl unterschiedlicher Prozessschritte wird reduziert und dadurch die Robustheit des gesamten Verfahrens erhöht, bzw. die Fehleranfälligkeit deutlich reduziert.

### Beispiel 11: (nicht erfindungsgemäß: Zumischung des Zuckers, des Allyl-PEG und des Allyl-CDs ohne Siliconisierung)

In 98,5 g des hydrophoben Organopolysiloxangel gemäß Beispiel 1 der vorliegenden Erfindung werden statt des Polyglucosidgruppen enthaltenden Polyorganosiloxans an gleicher Stelle folgende Mengen des Alkenylglucosids gemäß Beispiel 12 oder des polyoxyethylierten Allylakohols H₂C=CH-CH₂-(OC₂H₄)₁₀-OH gemäß Beispiel 6, zu beziehen unter der Bezeichnung Polyglycol A 500 bei Clariant oder des Allylcyclodextrins gemäß Beispiel 13 eingearbeitet:

| | |
|---|---|
| **Alkenylglucosid** | **1,5 g** |
| **polyoxyethylierter Allylalkohol** | **1,5 g** |
| **Allylcyclodextrin** | **1,5 g** |

Die erhaltenen Zubereitungen sind alle trüb.

In die erhaltenen Zubereitungen wird Wasser eingearbeitet analog der Vorgehensweise wie für Beispiel 1 beschrieben.

Dabei erweisen sie sich als instabil, die Mengen an Wasser, die sich einarbeiten ließen, sind in nachfolgender Tabelle aufgeführt:

| | Beispiel 1 mit (3a) | Beispiel 1 ohne (3a) + Alkylenglucosid | Beispiel 1 ohne (3a) + polyoxyethylierter Allylalkohol | Beispiel 1 ohne (3a) + Allylcyclodextrin |
|---|---|---|---|---|
| Gel:Wasser | 1 : 2,45 | 1 : <0,1 | 1 : <0,1 | 1 : 0,1 |

### Beispiel 12: Herstellung des Alkenylglucosids für die Synthese des Polyglucosidreste aufweisenden Polyorganosiloxans (3b)

Das Alkenylglucosid zur Herstellung des Polyglusidreste enthaltenden Polyorganosiloxans, das hier in den Beispielen eingesetzt wurde, wurde hergestellt gemäß dem in US 5,831,080 in Beispiel 1 (A) (Spalte 8, Zeilen 23 - 43) beschriebenen Verfahren, mit dem Unterschied, dass das Lösemittel im letzten Schritt nicht vollständig abdestilliert wird, da dies zu einem bei Raumtemperatur glasartigen Material führen würde, sondern dass ein Lösemitteltausch auf 1,2-Propandiol durchgeführt wird. Das Produkt ist eine ca. 50%-ige Lösung des Alkenylglucosids in 1,2-Propandiol.

### Beispiel 13: Herstellung des Allylcyclodextrins

Die Herstellung des Allylcyclodextrins erfolgt gemäß WO2020139403A1 Vorschrift "Synthesis of allyl modified methyl β-cyclodextrin" [0054 - 0055].

### Beispiel 14: Herstellung eines Polyglucosidreste aufweisenden Polyorganosiloxans (3b)

In ein 20 l Glasrührwerk mit Dosier- und Destilliereinrichtungen und dampfbetriebenem Heizmantel werden 2235 g (Me₃SiO_{1/2}) (Me₂SiO_{2/2})ₐ(MeHSiO_{2/2})_{b}(Me₃SiO_{1/2}) zugegeben, mit a:b = 9:1 und einer Kettenlänge von a+b = 60 Wiederholungseinheiten vorgelegt und auf 100°C vorgeheizt. Dabei wird mit einer Geschwindigkeit von 70 Umdrehungen pro Minute gerührt. In diese Vorlage werden 789 g einer Zubereitung aus der Alkenylglucosidlösung aus Beispiel 12, Hexachloroplatinsäure in Isopropanol, wässriger Kaliumhydroxidlösung innerhalb von 6 Stunden zudosiert. Die Zubereitung wird wie folgt hergestellt. Man erwärmt 789 g der Alkenylglucosidlösung gemäß Beispiel 12 in einem Wärmeschrank auf 60°C. Von der erwärmten Lösung werden 10 ml abgenommen und mit 0,224 g einer 50%-ige wässrigen Lösung von Kaliumhydroxid gemischt. Diese Mischung wird wieder in die restliche Alkenylglucosidlösung gegeben und gut vermischt. 0,18 g Hexachloroplatinsäure werden in 18,61 g Isopropanol gelöst. Diese Lösung wird in die Mischung aus Kaliumhydroxid und Alkenylglucosidlösung gegeben und gut vermischt.

Die entstandene Zubereitung wird wie oben beschrieben dosiert. Während der Dosierung wird die Heizung abgestellt.

Nach Dosierende wird eine Lösung aus 0,093 g Hexachloroplatinsäure und 9,3 g Isopropanol in das Reaktionsgefäß zugegeben und vermischt. Man erwärmt auf 120°C Innentemperatur und hält für 2 h bei dieser Temperatur. Anschließend wird die Heizung abgestellt und auf 110°C abgekühlt. Man dosiert 29 g Octadec-1-en zu ohne Heizen zu. Die Reaktionsmischung erwärmt sich leicht auf 113°C. Man erwärmt wieder auf 120°C und hält für 2 h bei dieser Temperatur. Anschließend wird auf 60°C abgekühlt.

Danach werden 6,51 kg eines linearen Polydimethylsiloxans mit einer Viskosität von 5 mPas zügig zugegeben und gut gemischt. Man erhöht die Temperatur im Rührwerk auf 130°C und legt Vakuum an. Man destilliert eine Mischung aus Isopropanol, 1,2-Propandiol und niedrigsiedenden Siloxananteilen solange aus, bis ein Endvakuum von 5 mbar erreicht wird.

Wenn bei diesen Bedingungen kein Destillat mehr erhalten wird, wird das Vakuum gebrochen und auf 30°C abgekühlt. Man gibt anschließend 22g einer 35%-igen Wasserstoffperocidlösung zu, heizt auf 60°C auf und hält für 1 h bei dieser Temperatur. Danach wird auf 23°C abgekühlt und der Inhalt des Reaktionsgefäßes über einen 25 µm Filter filtriert, so dass 8,7 kg eines klaren im Wesentlichen farblosen, höchstens sehr leicht gelblichen Filtrats erhalten werden.

Diese Zubereitung wird in der erhaltenen Form als Polyglucosidreste enthaltendes Polyorganosiloxan (3b) eingesetzt. Alle Einsatzmengen in den Beispielen beziehen sich auf diese Zubereitung.

**Der enthaltende Wirkstoff hat aufgrund seiner Zusammensetzung einen HLB-Wert von ca. 7. In der Zubereitung sind 30 Gewichtsprozent davon enthalten.**

### Beispiel 15: Herstellung des PEG Öls

Die Herstellung des Polyoxyethyleneinheiten enthaltenden Poyorganosiloxans erfolgt analog zur Vorgehensweise, wie sie in Beispiel 14 beschrieben ist.

Im Unterschied zu Beispiel 14 wird anstelle des Alkenylglucosids in 1,2-Propandiol gemäß Beispiel 12 hier der polyoxyethylierte Allylakohol H₂C=CH-CH₂-(OC₂H₄)₁₀-OH eingesetzt. Anstelle von 789 g des Alkenylglucosids in 1,2-Propandiol, die 394,5 g Alkenylglucosid enthalten, werden hier 1420 g aus 710 g des polyoxylierten Allylalkohols gemischt mit 710 g 1,2-Propandiol eingesetzt. Diese Mischung wird bei Raumtemperatur mit der gleichen Menge Hexachloroplatinsäure in Isopropanol gemischt wie in Beispiel 14 angegeben. Es wird keine wässrige Kaliumhydroxidlösung verwendet. Ansonsten bleiben die Vorlage- und Dosiermengen sowie die Dosierzeiten und Temperaturen die gleichen.

Auch die nachzudosierende Menge Hexachloroplatinsäure in Isopropanol ist die gleiche. Diese Synthese endet nach der Hydrosilylierung des Octadec-1-ens. D.h. der Bleichschritt mit Wasserstoffperoxid wird hier nicht durchgeführt. Man füllt nach der Destillation wie oben beschrieben über einen 25 µm Filter ab und erhält eine klare, farblose Lösung, die ca. 32 Gewichtsprozent des Polyglykolreste enthaltenden Polyorganosiloxanwirkstoffes gelöst in dem linearen Polyorganosiloxan mit einer Viskosität von 5 mPas enthält.

### Beispiel 16: Herstellung des CD-Öls

Die Herstellung des Cyclodextrinreste enthaltenden Poyorganosiloxans (3c) erfolgt analog zur Vorgehensweise, wie sie in Beispiel 14 beschrieben ist.

Im Unterschied zu Beispiel 14 wird anstelle des Alkenylglucosids in 1,2-Propandiol gemäß Beispiel 12 hier das allylmodifizierte β-Cyclodextrin gemäß Beispiel 13 eingesetzt.

Zur Hydrosilylierung werden 394,5 g einer 50%-igen Lösung des β-Cyclodextrins gemäß Beispiel 13 in 1,2-Propandiol verwendet. Ansonsten werden die Schritte gemäß Vorgehensweise nach Beispiel 14 ausgeführt.

Man erhält eine ca. 30 Gewichtsprozentige Lösung des Cyclodextrinreste enthaltenden Polyorganosiloxans (3c) in dem linearen Polydimethylsiloxan mit der Viskosität von 5 mPas.

## Patentansprüche

1. Hydrophile Organopolysiloxangelzubereitung enthaltend 2 bis 80 Gew.-% einer Mischung (X) aus
- 80 bis 99,9 Gew.-% mindestens eines hydrophoben Organopolysiloxangels hergestellt aus der Umsetzung entweder eines ungesättigten Organopolysiloxanharzes (1a) oder eines ungesättigten Organopolysiloxans (1b) oder eines Diens (1c) mit einem Si-H-haltigen Organopolysiloxan (2a) gegebenenfalls im Gemisch mit einem Si-H-haltigen Organopolysiloxan (2b) in Gegenwart eines die Anlagerung von Si-gebundenem Wasserstoff an aliphatische Mehrfachbindung fördernden Katalysatoren (K) (=Hydrosilylierungskatalysator) in Anwesenheit eines Verdünnungsmittels (4), wobei die Reaktion durch Zusatz einer als Katalysatorgift eingesetzten Stopperverbindung (5) gestoppt wird, die im hydrophoben Organopolysiloxangel verbleibt,
- 0,1 bis 20 Gew.-% mindestens einer Verbindung (3) ausgewählt aus der Gruppe enthaltend
(3a) Glycosidreste aufweisende Organopolysiloxane der allgemeinen Formel (V),
RₕR³ᵢSiO_{(4-h-i)/2} (V),
wobei
R gleich oder verschieden sein kann und einen einwertigen, gegebenenfalls substituierten Kohlenwasserstoffrest mit 1 bis 18 Kohlenstoffatomen je Rest bedeutet, wobei Heteroatome und -gruppen ausgeschlossen sind, die eine Hydrosilylierungsreaktion durch negative Einwirkung auf den Katalysator behindern würden,
R³ gleich oder verschieden sein kann und einen Rest der Formel
Z- (R⁴O)ⱼ-R⁵- (Va)
bedeutet, worin
Z einen Glycosidrest bedeutet, der aus 1 bis 10 Monosaccharideinheiten, vorzugsweise Glucoseeinheiten, aufgebaut ist,
R⁴ gleich oder verschieden sein kann und einen zweiwertigen Kohlenwasserstoffrest mit 2 bis 4 C-Atomen bedeutet,
j 0 oder eine ganze Zahl von 1 bis 20 ist und
R⁵ gleich oder verschieden sein kann und einen zweiwertigen Kohlenwasserstoffrest mit 2 bis 12 C-Atomen bedeutet,
h und i jeweils 0, 1, 2 oder 3 bedeuten, mit der Maßgabe, dass h + i ≤ 3 und in mindestens einer Wiederholungseinheit i mindestens den Wert 1 hat;
(3b) Polyglykolresten aufweisenden Organopolysiloxanen der Formel (VI)
RₕR⁶ᵢSiO_{(4-h-i)/2} (VI),
wobei
R die oben angegebenen Bedeutung hat,
R⁶ gleich oder verschieden sein kann und einen Rest der Formel
P-R⁷-R⁸- (VIa)
bedeutet, worin
P einen polyoxyalkylierten Rest der Art - (OCₙH₂ₙ)ₘ-OH bedeutet,
R⁷ eine chemische Bindung oder ein zweiwertiger Kohlenwasserstoffrest mit 1 bis 10 Kohlenstoffatomen, vorzugsweise ein Rest der Formel -CH₂- ,
-CH(CH₃)- oder -C(CH₃)₂- , bevorzugt ein Rest der Formel -CH₂- , ist und
n eine ganze Zahl von 1 bis 4, bevorzugt 2 oder 3, ist und
m eine ganze positive Zahl, vorzugsweise von 1 bis 40, ist,
R⁸ einen zweiwertigen Kohlenwasserstoffrest mit 2 bis 12 C-Atomen bedeutet, vorzugsweise ein Rest der Formel -CH₂-CH₂-,
-CH₂-CH(CH₃)- oder -CH₂-C(CH₃)₂-, bevorzugt ein Rest der Formel -CH₂-CH₂- ist,
h und i jeweils die weiter oben angegebene Bedeutung haben;
(3c) Cyclodextrinresten aufweisenden Organopolysiloxanen der Formel (VII)
RₕR⁹ᵢSiO_{(4-h-i)/2} (VII),
wobei
R die oben angegebenen Bedeutungen haben kann,
R⁹ gleich oder verschieden sein kann und einen Rest der Formel
C-R¹⁰- (VIIa)
bedeutet, worin
C einen Cyclodextrinrest der Form 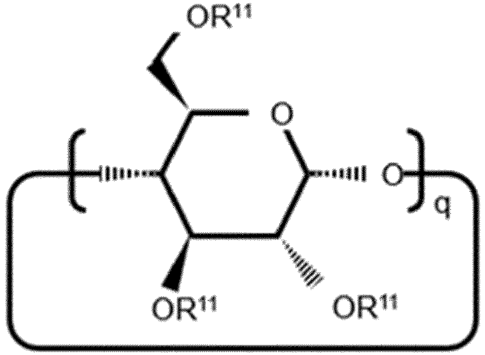
bedeutet,
in dem R¹¹ einen Wasserstoffrest oder einen Methylrest bedeutet, und
q die ganzen Zahlen 6 (alpha Cyclodextrin), 7 (beta Cyclodextrin) oder 8 (gamma Cyclodextrin) bedeutet,
R¹⁰ einen zweiwertigen gegebenenfalls mit Heteroatomen unterbrochenen Kohlenwasserstoffrest mit 2 bis 18 Kohlenstoffatomen bedeutet,
h und i jeweils die weiter oben angegebene Bedeutung haben;
wobei die Summe der Mischung (X) stets 100 Gew.% ergibt;
und 20 bis 98 Gew.-% mindestens eines Verdünnungsmittel (4) zur Viskositätseinstellung, welches verschieden ist von (3a), (3b) und (3c);
wobei die hydrophile Organopolysiloxangelzubereitung eine Steigerung der Wasseraufnahmefähigkeit um mindestens den Faktor 10 aufweist, verglichen mit dem eingesetzten hydrophoben Organopolysiloxangel;
und wobei die Summe aus der Mischung (X) und dem Verdünnungsmittel (4) stets 100 Gew.-% der hydrophilen Organopolysiloxangelzubereitung ergibt.

2. Hydrophile Organopolysiloxangelzubereitung gemäß Anspruch 1, **dadurch gekennzeichnet, dass** die Verbindung (3) nur ausgewählt wird aus der Gruppe der Verbindungen (3a).

3. Hydrophile Organopolysiloxangelzubereitung gemäß Anspruch 1, **dadurch gekennzeichnet, dass** die Verbindung (3) nur ausgewählt wird aus der Gruppe der Verbindungen (3b).

4. Hydrophile Organopolysiloxangelzubereitung gemäß Anspruch 1, **dadurch gekennzeichnet, dass** die Verbindung (3) nur ausgewählt wird aus der Gruppe der Verbindungen (3c).

5. Hydrophile Organopolysiloxangelzubereitung gemäß einem der Ansprüche 1 bis 4, **dadurch gekennzeichnet, dass** das hydrophobe Organopolysiloxangel ausgewählt wird aus solchen welche durch folgende Umsetzung erhalten werden:
- Reaktion von ungesättigten Organopolysiloxanharzen (1a) mit Si-H-funktionellen Vernetzern (2a), in Anwesenheit oder Abwesenheit von Si-H-haltigen Organopolysiloxanen (2b) und in Gegenwart eines Katalysators (K),
- Stoppen der Reaktion durch Zusatz einer als Katalysatorgift eingesetzten Stopperverbindung (5).

6. Kosmetische Zubereitung oder Zubereitung für die Körper- und Gesundheitspflege, Wasch- und Reinigungszubereitung, Zubereitung zur langanhaltenden Beduftung oder Insektenabwehr, enthaltend die hydrophile Organopolysiloxangelzubereitung gemäß einem der Ansprüche 1 bis 5.

7. Verfahren zur Herstellung einer hydrophilen Organopolysiloxangelzubereitung gemäß Anspruch 1, **dadurch gekennzeichnet, dass** die im Anspruch 1 genannten Komponenten miteinander vermengt werden.

8. Verwendung der hydrophilen Organopolysiloxangelzubereitung gemäß einem der Ansprüche 1 bis 5 in kosmetischen Zubereitungen oder Zubereitungen für die Körper- und Gesundheitspflege oder in Wasch- und Reinigungsprodukten oder in Produkten zur langanhaltenden Beduftung oder Insektenabwehr.

9. Verwendung der hydrophilen Organopolysiloxangelzubereitung gemäß einem der Ansprüche 1 bis 5 als Füllstoff oder Isolierungsmaterial für elektrische Kabel, Boden- oder Wasserbarrieren für Bodenstabilisierung oder als Ersatz für Epoxymaterialen, die in Bauteilen in der elektronischen Industrie verwendet werden.

## Claims

1. Hydrophilic organopolysiloxane gel preparation comprising 2 to 80 wt% of a mixture (X) of
- 80 to 99.9 wt% of at least one hydrophobic organopolysiloxane gel prepared from the reaction either of an unsaturated organopolysiloxane resin (1a) or of an unsaturated organopolysiloxane (1b) or of a diene (1c) with an Si-H-containing organopolysiloxane (2a) optionally in the mixture with an Si-H-containing organopolysiloxane (2b) in the presence of a catalyst (K) which promotes the addition of Si-bonded hydrogen onto aliphatic multiple bond (i.e. hydrosilylation catalyst) in the presence of a diluent (4), the reaction being stopped by addition of a stopper compound (5) which is used as a catalyst poison and which remains in the hydrophobic organopolysiloxane gel,
- 0.1 to 20 wt% of at least one compound (3) selected from the group containing
(3a) organopolysiloxanes containing glycoside radicals and of the general formula (V),
RₕR³ᵢSiO_{(4-h-i)/2} (V),
where
R may be identical or different and is a monovalent, optionally substituted hydrocarbon radical having 1 to 18 carbon atoms per radical, with exclusion of heteroatoms and hetero groups which would hinder a hydrosilylation reaction by adversely affecting the catalyst,
R³ may be identical or different and is a radical of the formula
Z-(R⁴O)ⱼ-R⁵- (Va), in which
Z is a glycoside radical composed of 1 to 10 monosaccharide units, preferably glucose units,
R⁴ may be identical or different and is a divalent hydrocarbon radical having 2 to 4 carbon atoms,
j is 0 or an integer from 1 to 20, and
R⁵ may be identical or different and is a divalent hydrocarbon radical having 2 to 12 carbon atoms,
h and i are each 0, 1, 2 or 3, with the proviso that h + i ≤ 3 and in at least one repeating unit i has a value of at least 1;
(3b) organopolysiloxanes containing polyglycol radicals and of the formula (VI)
RₕR⁶ᵢSiO_{(4-h-i)/2} (VI),
where
R has the definition indicated above,
R⁶ may be identical or different and is a radical of the formula
P-R⁷-R⁸- (VIa),
in which
P is a polyoxyalkylated radical of the type -(OCₙH₂ₙ)ₘ-OH,
R⁷ is a chemical bond or a divalent hydrocarbon radical having 1 to 10 carbon atoms, preferably a radical of the formula -CH₂-, -CH(CH₃)- or -C(CH₃)₂-, more preferably a radical of the formula -CH₂-, and
n is an integer from 1 to 4, preferably 2 or 3, and
m is a positive integer, preferably from 1 to 40,
R⁸ is a divalent hydrocarbon radical having 2 to 12 carbon atoms, preferably a radical of the formula -CH₂-CH₂-,
-CH₂-CH(CH₃)- or -CH₂-C(CH₃)₂-, more preferably a radical of the formula -CH₂-CH₂-,
h and i each have the definition indicated earlier on above;
(3c) organopolysiloxanes containing cyclodextrin radicals and of the formula (VII)
RₕR⁹ᵢSiO_{(4-h-i)/2} (VII),
where
R may have the definitions indicated above,
R⁹ may be identical or different and is a radical of the formula
C-R¹⁰- (VIIa),
in which 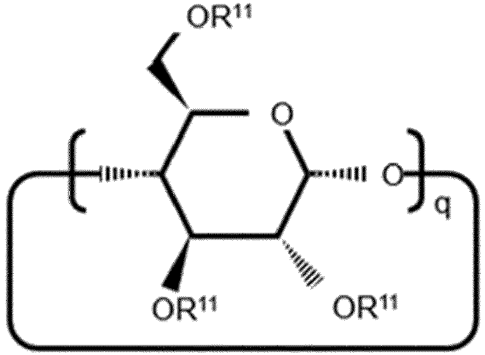
C is a cyclodextrin radical of the form
in which R¹¹ is a hydrogen radical or a methyl radical, and
q is the integers 6 (alpha cyclodextrin), 7 (beta cyclodextrin) or 8 (gamma cyclodextrin),
R¹⁰ is a divalent hydrocarbon radical having 2 to 18 carbon atoms which is optionally interrupted with heteroatoms,
h and i each have the definition indicated earlier on above;
where the sum of the mixture (X) always makes 100 wt%;
and 20 to 98 wt% of at least one diluent (4) for viscosity adjustment, which is different from (3a), (3b) and (3c);
where the hydrophilic organopolysiloxane gel preparation exhibits an increase in the water absorption capacity by at least a factor of 10, compared with the hydrophobic organopolysiloxane gel used;
and where the sum of the mixture (X) and the diluent (4) always makes 100 wt% of the hydrophilic organopolysiloxane gel preparation.

2. Hydrophilic organopolysiloxane gel preparation according to Claim 1, **characterized in that** the compound (3) is only selected from the group of the compounds (3a).

3. Hydrophilic organopolysiloxane gel preparation according to Claim 1, **characterized in that** the compound (3) is only selected from the group of the compounds (3b).

4. Hydrophilic organopolysiloxane gel preparation according to Claim 1, **characterized in that** the compound (3) is only selected from the group of the compounds (3c).

5. Hydrophilic organopolysiloxane gel preparation according to any of Claims 1 to 4, **characterized in that** the hydrophobic organopolysiloxane gel is selected from those obtained by the following reaction:
- reaction of unsaturated organopolysiloxane resins (1a) with Si-H-functional crosslinkers (2a), in the presence or absence of Si-H-containing organopolysiloxanes (2b) and in the presence of a catalyst (K),
- stopping of the reaction by addition of a stopper compound (5) used as a catalyst poison.

6. Cosmetic preparation or preparation for body care and health care, washing and cleaning preparation, preparation for long-lasting fragrancing or insect repellency, comprising the hydrophilic organopolysiloxane gel preparation according to any of Claims 1 to 5.

7. Method for producing a hydrophilic organopolysiloxane gel preparation according to Claim 1, **characterized in that** the components stated in Claim 1 are combined with one another.

8. Use of the hydrophilic organopolysiloxane gel preparation according to any of Claims 1 to 5 in cosmetic preparations or preparations for body care and health care, or in washing and cleaning products, or in products for long-lasting fragrancing or insect repellency.

9. Use of the hydrophilic organopolysiloxane gel preparation according to any of Claims 1 to 5 as a filler or insulating material for electrical cables, soil or water barriers for soil stabilization, or as substitute for epoxy materials which are used in components in the electronics industry.

## Revendications

1. Préparation de gel hydrophile d'organopolysiloxane, contenant 2 à 80% en poids d'un mélange (X) constitué par
- 80 à 99,9% en poids d'au moins un gel hydrophobe d'organopolysiloxane préparé à partir de la transformation soit d'une résine d'organopolysiloxane insaturé (1a), soit d'un organopolysiloxane insaturé (1b), soit d'un diène (1c) avec un organopolysiloxane contenant Si-H (2a), le cas échéant en mélange avec un organopolysiloxane contenant Si-H (2b), en présence d'un catalyseur favorisant une addition d'hydrogène lié par Si sur une liaison multiple aliphatique (K) (= catalyseur d'hydrosilylation) en présence d'un diluant (4), la réaction état arrêtée par addition d'un composé de blocage (5) utilisé en tant que poison de catalyseur, qui reste dans le gel hydrophobe d'organopolysiloxane,
- 0,1 à 20% en poids d'au moins un composé (3), choisi dans le groupe contenant
(3a) des organopolysiloxanes présentant des radicaux glycoside de formule générale (V),
RₕR³ᵢSiO_{(4-h-i)/2} (V),
dans laquelle
R peut être identique ou différent et signifie un radical hydrocarboné monovalent, le cas échéant substitué, comprenant 1 à 18 atomes de carbone par radical, des hétéroatomes et des hétérogroupes, qui empêcheraient une réaction d'hydrosilylation par action négative sur le catalyseur, étant exclus
R³ peut être identique ou différent et signifie un radical de formule
Z-(R⁴O)ⱼ-R⁵- (Va)
dans laquelle
Z signifie un radical glycoside qui est constitué par 1 à 10 motifs de monosaccharide, de préférence des motifs de glucose,
R⁴ peut être identique ou différent et signifie un radical hydrocarboné bivalent comprenant 2 à 4 atomes de carbone,
j vaut 0 ou un nombre entier de 1 à 20 et
R⁵ peut être identique ou différent et signifie un radical hydrocarboné bivalent comprenant 2 à 12 atomes de carbone,
h et i signifient à chaque fois 0, 1, 2 ou 3, sous réserve que h + i ≤ 3 et que, dans au moins un motif récurrent, i présente au moins la valeur 1 ;
(3b) des organopolysiloxanes présentant des radicaux polyglycol de formule (VI)
RₕR⁶ᵢSiO_{(4-h-i)/2} (VI),
dans laquelle
R présente la signification susmentionnée,
R⁶ peut être identique ou différent et signifie un radical de formule
P-R⁷-R⁸- (VIa)
dans laquelle
P signifie un radical polyoxyalkylé de type -(OCₙH₂ₙ)ₘ-OH,
R⁷ représente une liaison chimique ou un radical hydrocarboné bivalent comprenant 1 à 10 atomes de carbone, de préférence un radical de formule -CH₂-, -CH(CH₃)- ou -C(CH₃)₂-, de préférence un radical de formule -CH₂-, et
n représente un nombre entier de 1 à 4, de préférence 2 ou 3 et
m représente un nombre positif, de préférence de 1 à 40,
R⁸ signifie un radical hydrocarboné bivalent comprenant 2 à 12 atomes de carbone, de préférence un radical de formule -CH₂-CH₂-, -CH₂-CH(CH₃)- ou -CH₂-C(CH₃)₂-, de préférence un radical de formule - CH₂-CH₂-,
h et i présentent à chaque fois les significations indiquées ci-dessus ;
(3c) des organopolysiloxanes présentant des radicaux cyclodextrine de formule (VII)
RₕR⁹ᵢSiO_{(4-h-i)/2} (VII),
dans laquelle
R peut présenter les significations susmentionnées,
R⁹ peut être identique ou différent et signifie un radical de formule
C-R¹⁰- (VIIa)
dans laquelle
C signifie un radical cyclodextrine de formule 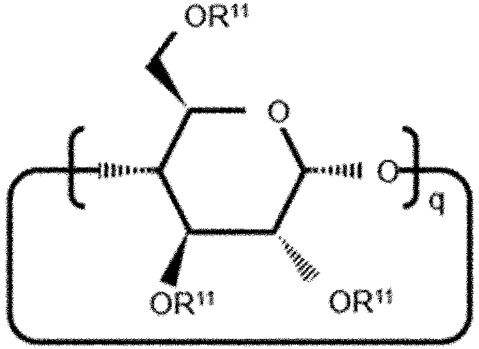 dans laquelle R¹¹ signifie un radical d'hydrogène ou un radical méthyle et
q signifie les nombres entiers 6 (alpha-cyclodextrine), 7 (bêta-cyclodextrine) ou 8 (gamma-cyclodextrine),
R¹⁰ signifie un radical hydrocarboné bivalent comprenant 2 à 18 atomes de carbone, le cas échéant interrompu par des hétéroatomes,
h et i présentent à chaque fois les significations indiquées ci-dessus ;
la somme du mélange (X) valant toujours 100% en poids ;
et 20 à 98 Gew-% d'au moins un diluant (4) pour le réglage de la viscosité, qui est différent de (3a), (3b) et (3c) ;
la préparation de gel hydrophile d'organopolysiloxane présentant une augmentation de l'aptitude à l'absorption d'eau d'au moins le facteur 10, par rapport au gel hydrophobe d'organopolysiloxane utilisé ;
et la somme du mélange (X) et du diluant (4) valant toujours 100 % en poids de la préparation de gel hydrophile d'organopolysiloxane.

2. Préparation de gel hydrophile d'organopolysiloxane selon la revendication 1, **caractérisée en ce que** le composé (3) est uniquement choisi dans le groupe des composés (3a).

3. Préparation de gel hydrophile d'organopolysiloxane selon la revendication 1, **caractérisée en ce que** le composé (3) est uniquement choisi dans le groupe des composés (3b).

4. Préparation de gel hydrophile d'organopolysiloxane selon la revendication 1, **caractérisée en ce que** le composé (3) est uniquement choisi dans le groupe des composés (3c).

5. Préparation de gel hydrophile d'organopolysiloxane selon l'une quelconque des revendications 1 à 4, **caractérisée en ce que** le gel hydrophobe d'organopolysiloxane est choisi parmi ceux obtenus par la transformation suivante :
- réaction de résines d'organopolysiloxane insaturé (1a) avec des réticulants à fonctionnalité Si-H (2a), en présence ou en l'absence d'organopolysiloxanes contenant Si-H (2b) et en présence d'un catalyseur (K),
- arrêt de la réaction par addition d'un composé de blocage utilisé en tant que poison de catalyseur (5).

6. Préparation cosmétique ou préparation pour le soin corporel et de santé, préparation de lavage et de nettoyage, préparation pour le parfum durable ou la répulsion durable d'insectes, contenant la préparation de gel hydrophile d'organopolysiloxane selon l'une quelconque des revendications 1 à 5

7. Procédé pour la confection d'une préparation de gel hydrophile d'organopolysiloxane selon la revendication 1, **caractérisé en ce que** les composants mentionnés dans la revendication 1 sont mélangés les uns avec les autres.

8. Utilisation de la préparation de gel hydrophile d'organopolysiloxane selon l'une quelconque des revendications 1 à 5 dans des préparations cosmétiques ou des préparations pour le soin corporel et de santé ou dans des produits de lavage de nettoyage ou dans des produits pour le parfum durable ou la répulsion durable d'insectes.

9. Utilisation de la préparation de gel hydrophile d'organopolysiloxane selon l'une quelconque des revendications 1 à 5 comme charge ou matériau d'isolation pour des câbles électriques, barrières de sol ou à l'eau pour la stabilisation du sol ou comme produit de remplacement de matériaux époxy qui sont utilisés dans des composants dans l'industrie électronique.
